# EUROPEAN PATENT SPECIFICATION

(11) **EP 2 558 866 B1**
(45) Date of publication and mention of the grant of the patent: **17.08.2016**
(21) Application number: 11769710.2
(22) Date of filing: 15.04.2011
(51) Int. Cl.: G01N 33/574, A61K 31/137, A61P 35/00, A61P 35/02, A61K 45/06

(54) **POTENTIATION OF ANTI-CANCER ACTIVITY THROUGH COMBINATION THERAPY WITH BER PATHWAY INHIBITORS**
POTENZIERUNG EINER ANTIKREBS-WIRKUNG DURCH EINE KOMBINATIONSTHERAPIE MIT BER-PFAD-HEMMERN
POTENTIALISATION DE L'ACTIVITÉ ANTICANCÉREUSE PAR POLYTHÉRAPIE AVEC DES INHIBITEURS DE LA VOIE BER

(30) Priority: 15.04.2010 US 324658 P
(43) Date of publication of application: 20.02.2013
(73) Proprietor: Tracon Pharmaceuticals, Inc., San Diego, CA 92122 (US)
(72) Inventor: THEUER, Charles, Carlsbad CA 92009 (US)
(74) Representative: Cole, William Gwyn
(86) International application number: PCT/US2011/032762
(87) International publication number: WO 2011/130689

(56) References cited:
- US-A1- 2002 198 264
- US-A1- 2008 234 298
- US-A1- 2009 029 966
- US-A1- 2010 003 192
- T. J. KINSELLA: "Coordination of DNA Mismatch Repair and Base Excision Repair Processing of Chemotherapy and Radiation Damage for Targeting Resistant Cancers", CLINICAL CANCER RESEARCH, vol. 15, no. 6, 10 March 2009 (2009-03-10) , pages 1853-1859, XP055077944, ISSN: 1078-0432, DOI: 10.1158/1078-0432.CCR-08-1307
- PALMA JOANN P ET AL: "The PARP Inhibitor, ABT-888 Potentiates Temozolomide: Correlation with Drug Levels and Reduction in PARP Activity In Vivo", ANTICANCER RESEARCH, vol. 28, no. 5A, September 2008 (2008-09), pages 2625-2635, XP002712423, ISSN: 0250-7005
- J. P. PALMA ET AL: "ABT-888 Confers Broad In vivo Activity in Combination with Temozolomide in Diverse Tumors", CLINICAL CANCER RESEARCH, vol. 15, no. 23, 24 November 2009 (2009-11-24), pages 7277-7290, XP055078012, ISSN: 1078-0432, DOI: 10.1158/1078-0432.CCR-09-1245
- BULGAR ALINA D ET AL: "Abstract n° 682: Dual inhibition of BER by TRC102 and PARP inhibitor (ABT 888) synergistically enhances cytotoxicity of TMZ in human melanoma", Proceedings of the 101st Annual Meeting of the American Association for Cancer Research (17-21 Apr. 2010) Cancer Research, vol. 70, no. 8, Suppl. 1 15 April 2010 (2010-04-15), XP002712424, DOI: 10.1158/1538-7445.AM10-682 Retrieved from the Internet: URL:http://cancerres.aacrjournals.org/cgi/ content/meeting_abstract/70/8_MeetingAbstr acts/682 [retrieved on 2013-09-06]

## Description

### FIELD OF THE INVENTION

The disclosure generally relates to pharmaceutical compositions and methods for the treatment of certain cancers. Provided herein are pharmaceutical compositions and methods of treating cancer wherein the cytotoxic activity of an anticancer agent or radiation therapy is potentiated by the combination with base excision repair (BER) pathway inhibitors, such as methoxyamine and PARP inhibitors.

### BACKGROUND OF THE INVENTION

Cancer is a worldwide problem. As such, finding compositions and methods for the treatment of cancer is of vital interest. The treatment of cancer falls into three general categories: chemotherapy, radiation therapy, and surgery. Frequently, therapies are combined since a combination of therapies often increases the probability that the cancer will be eradicated as compared to treatment strategies utilizing a single therapy. Most typically, the surgical excision of large tumor masses is followed by chemotherapy and/or radiation therapy.

Chemotherapeutic anticancer agents work in a number of ways. For example, anticancer agents work by interfering with cell cycle progression or by generating DNA strand breaks. If the cancer cell is not able to overcome the cell cycle blockage or cell injury, the cell will often die via apoptotic mechanisms. However, in certain instances cancer cells develop resistance to anticancer agents, which results either in the renewed spread of the cancer and/or the requirement for higher dosages of the anticancer agent, which in some instances is toxic to the patient.

Melanoma, the most fatal skin cancer, has increased in incidence by 15-fold in the past 40 years; the fastest rate increase of any human malignancy. This disease metastasizes rapidly and is highly resistant to chemotherapy. Temozolomide (TMZ) is an important part of treatment regimens for advanced metastatic melanoma. However, drug resistance often results in treatment failure. A major resistance factor is the presence of elaborate mechanisms of DNA repair. US2002/0198264 discloses that temozolomide (TMZ) and methoxyamine may be used as a treatment for certain tumors that are resistant to treatment with TMZ alone. It further discloses that TMZ and a PARP inhibitor may be used as a treatment for certain tumors that are resistant to treatment with TMZ alone.

### SUMMARY

The present invention provides a) Methoxyamine; b) veliparib; and c) temozolomide; for simultaneous, separate or sequential use in treating cancer, wherein the methoxyamine and veliparib potentiate the cytotoxic activity of the temozolomide.

Described herein are novel pharmaceutical compositions and methods of treatments that synergistically potentiate the cytotoxicity of an anticancer agent or radiation therapy by combining the anticancer agent or radiation therapy with at least two base excision repair (BER) pathway inhibitors, such as methoxyamine and a PARP inhibitor.

Described herein is a pharmaceutical composition comprising (a) an anticancer agent selected from the group consisting of an alkylating agent and an antimetabolite, (b) methoxyamine (as a first base excision repair (BER) pathway inhibitor), and (c) a PARP inhibitor (as a second BER pathway inhibitor), wherein the methoxyamine and the PARP inhibitor potentiate the cytotoxic activity of the anticancer agent. In some cases, the methoxyamine and the PARP inhibitor synergistically potentiate the cytotoxic activity of said anticancer agent.

In some cases, the alkylating agent is selected from the group consisting of cyclophosphamide, chlorambucil, melphalan, chlormethine (mustine), ifosfamide, trofosfamide, prednimustine, bendamustine, busulfan, treosulfan, mannosulfan, thiotepa, triaziquone, carboquone, carmustine, lomustine, semustine, streptozocin, fotemustine, nimustine, ranimustine, etoglucid, mitobronitol, pipbroman, temozolomide (TMZ), and dacarbazine. In specific cases, the alkylating agent is TMZ or a pharmaceutically acceptable salt thereof. In some cases, the antimetabolite is selected from the group consisting of methotrexate, ralitrexed, pemetrexed, pralatrexate, mercaptopurine, azathioprine, thioguanine, clabridine, fludarabine, clofarabine, nelarabine, pentostatin, cytarabine, fluorouracil, floxuridine, tegafur, carmofur, gemcitabine, capecitabine, azacitidine, decitabine, fluorouracil combinations, and tegafur combinations. In specific cases, the antimetabolite is pemetrexed or a pharmaceutically acceptable salt thereof.

In some cases, the PARP inhibitor is selected from the group consisting of 4-iodo-3-nitrobenzamide, olaparib (AZD-2281; KU0059436), iniparib (BSI-201), veliparib (ABT-888), AG-014699, CEP9722, MK4827, INO-1001, E7016, AZD2461, LT-673, PD128763, and 3-aminobenzamide. In specific cases, the PARP inhibitor is ABT-888.

Described herein is a method of treating cancer, said method comprising administering to an individual in need thereof (a) an anticancer agent selected from the group consisting of an alkylating agent and an antimetabolite, (b) methoxyamine (as a first BER pathway inhibitor), and (c) a PARP inhibitor (as a second BER pathway inhibitor), wherein the methoxyamine and the PARP inhibitor potentiate the cytotoxic activity of the anticancer agent. In some cases, the methoxyamine and the PARP inhibitor synergistically potentiate the cytotoxic activity of the anticancer agent.

In some cases, the alkylating agent is selected from the group consisting of cyclophosphamide, chlorambucil, melphalan, chlormethine (mustine), ifosfamide, trofosfamide, prednimustine, bendamustine, busulfan, treosulfan, mannosulfan, thiotepa, triaziquone, carboquone, carmustine, lomustine, semustine, streptozocin, fotemustine, nimustine, ranimustine, etoglucid, mitobronitol, pipbroman, TMZ, dacarbazine. In specific cases, the alkylating agent is TMZ, or a pharmaceutically acceptable salt thereof. In certain cases, the antimetabolite is selected from the group consisting of methotrexate, ralitrexed, pemetrexed, pralatrexate, mercaptopurine, azathioprine, thioguanine, clabridine, fludarabine, clofarabine, nelarabine, pentostatin, cytarabine, fluorouracil, floxuridine, tegafur, carmofur, gemcitabine, capecitabine, azacitidine, decitabine, fluorouracil combinations, tegafur combinations. In specific cases, the antimetabolite is pemetrexed, or a pharmaceutically acceptable salt thereof.

In some cases, the PARP inhibitor is selected from the group consisting of 4-iodo-3-nitrobenzamide, olaparib (AZD-2281; KU0059436), iniparib (BSI-201), veliparib (ABT-888), AG-014699, CEP9722, MK4827, INO-1001, E7016, AZD2461, LT-673, PD128763, and 3-aminobenzamide. In specific cases, said PARP inhibitor is ABT-888.

In certain cases, the cancer is brain cancer, bladder cancer, breast cancer, cervical cancer, colon and rectal cancer, glioblastoma multiform, hepatocellular cancer, kidney (renal) cancer, leukemia, lung cancer, non-small-cell lung cancer, melanoma, mesothelioma, non-Hodgkin lymphoma, ovarian cancer, pancreatic cancer, prostate cancer, skin cancer (non-melanoma), thyroid cancer. In some cases, the cancer is lung cancer, non-small cell lung cancer, mesothelioma, brain cancer, glioblastoma multiforme, skin cancer, or melanoma. In specific cases, the cancer is melanoma.

In some cases, the anticancer agent, the methoxyamine, and the PARP inhibitor are administered orally, intravenously, intraperitoneally, directly by injection to a tumor, topically, or a combination thereof. In certain cases, the anticancer agent, the methoxyamine, and the PARP inhibitor are administered as a combination formulation. In some cases, the anticancer agent, the methoxyamine, and the PARP inhibitor are administered as individual formulations. In certain cases, the anticancer agent and the methoxyamine, the anticancer agent and the PARP inhibitor, or the methoxyamine and the PARP inhibitor are administered as a combination formulation. In some cases, the formulations are administered sequentially. In other cases, the formulations are administered simultaneously.

In certain cases, temozolomide is administered at doses of about 5 mg/m² per day, 10 mg/m² per day, 25 mg/m² per day, about 50 mg/m² per day, about 75 mg/m² per day, about 100 mg/m² per day, about 125 mg/m² per day, about 150 mg/m² per day, about 175 mg/m² per day, about 200 mg/m² per day, about 225 mg/m² per day, or about 250 mg/m² per day. In specific cases, temozolomide is administered at doses of about 25 mg/m² per day to about 200 mg/m² per day. In some cases, pemetrexed is administered at doses of about 100 mg/m² per day, about 125 mg/m² per day, about 150 mg/m² per day, about 175 mg/m² per day, about 200 mg/m² per day, about 225 mg/m² per day, about 250 mg/m² per day, about 275 mg/m² per day, about 300 mg/m² per day, about 325 mg/m² per day, about 350 mg/m² per day, about 375 mg/m² per day, about 400 mg/m² per day, about 425 mg/m² per day, about 450 mg/m² per day, about 475 mg/m² per day, about 500 mg/m² per day, about 525 mg/m² per day, about 550 mg/m² per day, about 600 mg/m² per day, or about 650 mg/m² per day. In specific cases, pemetrexed is administered at doses of about 200 mg/m² per day to about 500 mg/m² per day. In certain cases, methoxyamine is administered at doses of about 1 mg/m² per day, about 2 mg/m² per day, about 5 mg/m² per day, about 10 mg/m² per day, about 15 mg/m² per day, about 20 mg/m² per day, about 25 mg/m² per day, about 30 mg/m² per day, about 35 mg/m² per day, about 40 mg/m² per day, about 45 mg/m² per day, about 50 mg/m² per day, about 55 mg/m² per day, about 60 mg/m² per day, about 70 mg/m² per day, about 80 mg/m² per day, about 90 mg/m² per day, about 100 mg/m² per day. In specific cases, methoxyamine is administered at doses of about 5 mg/m² per day to about 100 mg/m² per day. In some cases, the PARP inhibitor is administered at doses of about 1 mg/kg per day, about 2 mg/kg per day, about 5 mg/kg per day, about 10 mg/kg per day, about 15 mg/kg per day, about 20 mg/kg per day, about 25 mg/kg per day, about 30 mg/kg per day, about 35 mg/kg per day, about 40 mg/kg per day, about 45 mg/kg per day, about 50 mg/kg per day, about 60 mg/kg per day, about 70 mg/kg per day, about 80 mg/kg per day, about 90 mg/kg per day, about 100 mg/kg per day, about 125 mg/kg per day, about 150 mg/kg per day, about 175 mg/kg per day, about 200 mg/kg per day, about 250 mg/kg per day, or about 300 mg/kg per day.

Described herein is a method of treating cancer, said method comprising administering to an individual in need thereof, (a) radiation therapy, (b) methoxyamine (as a first BER pathway inhibitor), and (c) a PAPR inhibitor (as a second BER pathway inhibitor), wherein the methoxyamine and the PARP inhibitor potentiate the effectiveness of the radiation therapy. In some cases, the methoxyamine and the PARP inhibitor synergistically potentiate the cytotoxic activity of the radiation therapy.

In certain cases, the PARP inhibitor is selected from the group consisting of 4-iodo-3-nitrobenzamide, olaparib (AZD-2281; KU0059436), iniparib (BSI-201), veliparib (ABT-888), AG-014699, CEP9722, MK4827, INO-1001, E7016, AZD2461, LT-673, PD128763, and 3-aminobenzamide. In specific cases, said PARP inhibitor is ABT-888.

In certain cases, the cancer is brain cancer, bladder cancer, breast cancer, cervical cancer, colon and rectal cancer, glioblastoma multiform, hepatocellular cancer, kidney (renal) cancer, leukemia, lung cancer, non-small-cell lung cancer, melanoma, mesothelioma, non-Hodgkin lymphoma, ovarian cancer, pancreatic cancer, prostate cancer, skin cancer (non-melanoma), thyroid cancer. In some cases, said cancer is lung cancer, non-small cell lung cancer, mesothelioma, brain cancer, glioblastoma multiforme, skin cancer, or melanoma. In specific cases, said cancer is melanoma.

In certain cases, the methoxyamine and the PARP inhibitor are administered orally, intravenously, intraperitoneally, directly by injection to a tumor, topically, or a combination thereof. In some cases, the methoxyamine and the PARP inhibitor are administered as a combination formulation. In certain cases, the methoxyamine and the PARP inhibitor are administered as individual formulations. In some cases, the radiation therapy and the formulations are administered sequentially. In other cases, the radiation therapy and the formulations are administered simultaneously.

In certain cases, methoxyamine is administered at doses of about 1 mg/m² per day, about 2 mg/m² per day, about 5 mg/m² per day, about 10 mg/m² per day, about 15 mg/m² per day, about 20 mg/m² per day, about 25 mg/m² per day, about 30 mg/m² per day, about 35 mg/m² per day, about 40 mg/m² per day, about 45 mg/m² per day, about 50 mg/m² per day, about 55 mg/m² per day, about 60 mg/m² per day, about 70 mg/m² per day, about 80 mg/m² per day, about 90 mg/m² per day, about 100 mg/m² per day. In specific cases, methoxyamine is administered at doses of about 5 mg/m² per day to about 100 mg/m² per day. In some cases, the PARP inhibitor is administered at doses of about 1 mg/kg per day, about 2 mg/kg per day, about 5 mg/kg per day, about 10 mg/kg per day, about 15 mg/kg per day, about 20 mg/kg per day, about 25 mg/kg per day, about 30 mg/kg per day, about 35 mg/kg per day, about 40 mg/kg per day, about 45 mg/kg per day, about 50 mg/kg per day, about 60 mg/kg per day, about 70 mg/kg per day, about 80 mg/kg per day, about 90 mg/kg per day, about 100 mg/kg per day, about 125 mg/kg per day, about 150 mg/kg per day, about 175 mg/kg per day, about 200 mg/kg per day, about 250 mg/kg per day, or about 300 mg/kg per day.

### BRIEF DESCRIPTION OF THE DRAWINGS

The invention herein is set forth in the appended claims. A better understanding of the features and advantages of the cases disclosed herein will be obtained by reference to the following detailed description that sets forth illustrative cases, in which the principles of the cases are utilized, and the accompanying drawings.
**Figure 1** illustrates the potentiation of cell death by combining a PARP inhibitor (ABT-888) with temozolomide (TMZ) and methoxyamine (MX) in A375 melanoma cells.
**Figure 2** illustrates the potentiation of cell death by combining a PARP inhibitor (ABT-888) with temozolomide (TMZ) and methoxyamine (MX) in WM9 melanoma cells.

### DETAILED DESCRIPTION OF THE INVENTION

Base Excision Repair (BER) is an important drug resistant factor because of the variety of its substrates and its ability to rapidly and efficiently repair DNA lesions. Repair of abasic [apurinic/apyrimidinic (AP)] sites is a crucial step in BER. It has been shown that in the BER pathway, the repair of an AP site proceeds much more quickly than the repair of a single base anomaly, such as an uracil or an 8-oxoguanine base. Thus, the effect of AP sites on cell death is minimal in the presence of efficient BER. Although AP sites potentially act as topoisomerase IIα (topo II) substrate, it is apparent that topo II cannot compete successfully against the BER machinery for processing of the DNA lesions. Therefore, only when the BER pathway is interrupted, do unrepaired AP sites become toxic.

Topo II is an essential enzyme that plays a critical role in many DNA processes, including DNA replication/recombination and chromosome segregation. To carry out its important physiologic functions, topo II alters DNA topology by passing an intact double helix through a transient double-stranded break in the genetic material. Thus, whereas the enzyme is necessary for cell survival, it also has the capacity to fragment the genome. During the double-stranded DNA passage reaction, topo II has preferential cleavage sites in the DNA. Its two active sites (tyrosyl residues) covalently bind to a 5 -phosphoryl group on each DNA strand, forming a topo II-cleavable DNA complex. Normally, these cleavage complexes are present at low levels and can be tolerated by cells. However, conditions that significantly increase the physiologic concentration of this cleavage complex will convert this physiologic process to a lethal toxicity. Many DNA lesions, such as abasic sites [apurininc/pyrimidinic (AP) sites], nicks, or smaller adducts, act as topo II substrates, of which AP sites seem to be the most active. When AP sites are located within topo II cleavage sites, they remarkably stimulate topo II-mediated DNA fragmentation. Therefore, AP sites are potentially lethal.

AP sites are the most common damage induced by alkylating therapeutic agents and formed as a consequence of removal of modified bases by DNA N-glycosylases. For instance, the anticancer agent temozolomide (TMZ) forms O⁶-methylguanine, N⁷-methylguanine, and N³-methyladenine DNA adducts. These DNA lesions are repaired by at least two mechanisms. First, for example, the O⁶-methylguan DNA adduct, a cytotoxic and genotoxic lesion, is repaired by O⁶-methylguan DNA-methyltransferase. Thus, O⁶-methylguan DNA-methyltransferase is a major mechanism of resistance to alkylating agents. Second, N⁷-methylguanine and N³-methyladenine DNA adducts are repaired by BER through the removal of these inappropriate bases by DNA glycosylases, generating AP sites in double-stranded DNA. AP sites, the toxic intermediates of BER, are subsequently recognized by AP endonucleases that incise the phosphodiester backbone immediately 5' to the lesion, leaving behind a strand break with a normal 3-hydroxyl group and an abnormal 5-abasic terminus. "Short-patch" BER proceeds with DNA polymerase removing the 5-abasic residue via its 5 -deoxyribose-phosphodiesterase activity and filling in the single nucleotide gap. To complete the process, DNA ligase I or a complex of XRCC1 and ligase III seals the nick. The cellular BER pathway is rapid and efficient, thus contributing to resistance to the therapeutic killing effect of TMZ.

### Methoxyamine as BER inhibitor

The role of base excision repair (BER) in conferring TMZ resistance has been explored in a combined therapy by targeting BER with methoxyamine (MX), an AP site binder and inhibitor of BER. MX binds to abasic AP sites and disrupts the BER pathway. MX has previously been studied as a structural modulator of AP sites that enhances the therapeutic effect of alkylating agents, such as TMZ, through its ability to block the repair of AP sites formed by TMZ. MX covalently binds to AP sites to form methoxyamine bound AP (MX-AP) sites, which are structurally modified AP sites. These MX-AP sites are resistant to recognition and repair by AP endonuclease, and the persistence of the lesions leads to cell death. The potentiation of TMZ by methoxyamine has been validated in different tumor types in vitro and in xenograft models. MX potentiation of TMZ is accompanied by a remarkable induction of DNA strand breaks.

Blockage of BER by MX improves the therapeutic efficacy of alkylating agents and antimetabolites. In xenograft studies, MX efficiently enhanced antitumor effect of TMZ in several colon cancer cell lines regardless of genetic status. Compared with TMZ alone, the combination of TMZ and MX had no demonstrable additive toxicity in nude mice carrying human tumor xenografts. The inhibition of tumor growth was associated with apoptotic death and chromosome aberrations in xenograft tumors after mice received treatment with TMZ and MX. In certain instances, MX-AP sites are considered to be the major lesions produced by the combination of TMZ and MX and are responsible for inducing DNA breakages by acting as substrates for topo II, with topo II-mediated DNA double-strand breaks then leading to cell death.

BER is a highly coordinated cellular biochemical system essential to cell survival. However, in some instances, variability of expression of BER proteins affects the dynamics of the repair pathway or change the repair rate that determines the cellular sensitivity to the cytotoxicity exerted by alkylating agents like TMZ. BER proteins are coordinately and differentially expressed in melanoma cell lines and, for example, are higher in A375 and much lower in WM164 cells. When these cells were treated with the combination of TMZ and MX, MX efficiently potentiated TMZ cytotoxicity by about 3 fold in A375, but failed to significantly enhance the killing effect of TMZ in WM164 cells. Similar results were observed in xenograft model as no significant enhancement of TMZ antitumor activity by MX was observed in mice carrying WM164 melanoma xenografts. In some instances, the failure of TMZ-potentiation by MX in WM164 cells is related to the low activity of BER proteins, particularly low levels of methylpurine-DNA glycosylase. This deficiency in BER proteins decreases the formation of AP sites, the targets of MX action, and therefore reduces the potentiation effect of MX when dosed with TMZ.

### PARP Inhibitors

Another protein involved in cellular processes related to DNA repair through BER and programmed cell death is poly(ADP-ribose)polymerase (PARP). PARP is a DNA nick-sensor that signals the presence of DNA damage and facilitates DNA repair. The polymerase catalyzes the addition of ADP-ribose units to DNA, histones, and various DNA repair enzymes, which affects cellular processes as diverse as replication, transcription, differentiation, gene regulation, protein degradation, and spindle maintenance.

Increased PARP activity is one of the mechanisms by which tumor cells avoid apoptosis caused by DNA-damaging agents. PARP is essential for the repair of single stranded DNA breaks (SSB) through the bases excision repair (BER) pathways. Inhibition of PARP sensitizes tumor cells to cytotoxic therapy (e.g. TMZ, platinums, topoisomerase I inhibitors, radiation), which induce DNA damage that would normally be repaired through the BER system. The inhibition of PARP inhibits BER with the accumulation of large numbers of unrepaired DNA strand breaks.

### Combination Therapy

Described herein are pharmaceutical compositions and methods of treatments that synergistically potentiate the effectiveness of anticancer therapy by combining the anticancer therapy with at least two base excision repair (BER) pathway inhibitors. In certain cases, the BER pathway inhibitors have a different mode of action. In some cases, BER pathway inhibitors bind to abasic AP sites and block BER (AP site binders). In certain cases, BER pathway inhibitors are PARP inhibitors. In some cases, the anticancer therapy is combined with two BER pathway inhibitors with a different mode of action. In certain cases, the anticancer therapy is combined with a BER pathway inhibitor, which is an AP site binder, and a BER pathway inhibitor, which is an inhibitor of PARP. In some cases, the BER pathway inhibitor is the AP site binder methoxyamine (MX). In some cases, the PARP inhibitor is selected from the group consisting of, but not limited to, 4-iodo-3-nitrobenzamide, olaparib (AZD-2281; KU0059436), iniparib (BSI-201), veliparib (ABT-888), AG-014699, CEP9722, MK4827, INO-1001, E7016, AZD2461, LT-673, PD128763, and 3-aminobenzamide. In specific cases, the PARP inhibitor is ABT-888.

In some cases, a pharmaceutical composition comprising anticancer therapy, methoxyamine, and a PARP inhibitor, potentiates the cytotoxic activity of said anticancer therapy. In certain cases, a pharmaceutical composition comprising anticancer therapy, methoxyamine and ABT-888 potentiates the cytotoxic activity of said anticancer therapy.

In some cases, the combination of MX and a PARP inhibitor potentiates the cytotoxic anti-tumor effect of anticancer therapy through dual inhibition of BER. In certain cases, the combination of MX and a PARP inhibitor synergistically potentiates the cytotoxic anti-tumor effect of anticancer therapy. Synergistically means that the resulting potentiation of the cytotoxicity of anticancer therapy is greater than just adding the potentiating effect that the individual BER have on anticancer therapy.

In some instances, potentiator agents are chemotherapeutic compounds that by themselves have no or only very limited anticancer activity, but they interfere with DNA repair mechanisms. In certain instances, if anticancer therapy is used in concert with one or more potentiator agents, the dosage of any single drug (e.g., chemotherapeutic anticancer agent) or therapy (e.g., radiation therapy) may be lowered. In some instances, this is beneficial to the patient since using lower levels of chemotherapeutic anticancer agents or radiation therapy is generally safer for the patient. In certain instances, cancer cells are less likely to generate resistance to the combination of treatments as they are to a single form of treatment.

In some instances, anticancer therapy is divided into three main categories: surgery, radiation therapy, and chemotherapy with anticancer agents. In certain instances, these therapies are combined to increase the probability of successful therapy and decrease the probability for the development of the cancer developing resistance to the anticancer therapy.

A large number of chemotherapeutic anticancer agents are available, which have been classified into different classes. In some cases, the anticancer therapy comprises chemotherapeutic anticancer agents. Examples of chemotherapeutic anticancer agents include alkylating agents, antimetabolites, plant alkaloids and other natural products, cytotoxic antibiotics and related substances, and other antineoplastic agents.

In some cases, the anticancer therapy comprises therapy with chemotherapeutic alkylating agents. Examples of alkylating agents include nitrogen mustards, alkyl sulfonates, ethylene imines, nitrosoureas, epoxides, and other alkylating agents.

In certain cases, the anticancer therapy comprises therapy with a nitrogen mustard alkylating agent. Examples of nitrogen mustard alkylating agents include cyclophosphamide, chlorambucil, melphalan, chlormethine (mustine), ifosfamide, trofosfamide, prednimustine, bendamustine. In some cases, the anticancer therapy comprises therapy with an alkyl sulfonate. Examples of alkyl sulfonates alkylating agents include busulfan, treosulfan, mannosulfan. In certain cases the anticancer therapy includes therapy with an ethylene imine alkylating agent. Examples of ethylene imine alkylating agents include thiotepa, triaziquone, carboquone. In some cases the anticancer therapy includes therapy with a nitrosourea alkylating agent. Examples of nitrosourea alkylating agents include carmustine, lomustine, semustine, streptozocin, fotemustine, nimustine, ranimustine. In certain cases the anticancer therapy includes therapy with an epoxide alkylating agent. Examples of epoxide alkylating agents include etoglucid. In certain cases the anticancer therapy includes therapy with other alkylating agents. Examples of other alkylating agents include mitobronitol, pipbroman, temozolomide (TMZ), dacarbazine. Additional example of other alkylating agents include uramustine, procarbazine, altretamine, mitozolomide.

In some cases, the anticancer therapy comprises therapy with chemotherapeutic antimetabolites. Examples of antimetabolites include folic acid analogs, purine analogs, and pyrimidine analogs.

In certain cases, the anticancer therapy comprises therapy with a folic acid analog antimetabolite. Examples of folic acid analog antimetabolites include methotrexate, ralitrexed, pemetrexed, pralatrexate. In some cases, the anticancer therapy comprises therapy with a purine analog antimetabolite. Examples of purine analog antimetabolites include mercaptopurine, thioguanine, clabridine, fludarabine, clofarabine, nelarabine. In certain cases, the anticancer therapy comprises therapy with a pyrimidine analog antimetabolite. Examples of pyrimidine analog antimetabolites include cytarabine, fluorouracil, tegafur, carmofur, gemcitabine, capecitabine, azacitidine, decitabine, fluorouracil combinations, tegafur combinations.

In some cases, the anticancer therapy comprises therapy with chemotherapeutic plant alkaloids and other natural products. Examples of plant alkaloids and other natural products include vinca alkaloids and analogs, podophyllotoxin derivatives, colchicine derivatives, taxanes, and other plant alkaloids and natural products.

In certain cases, the anticancer therapy comprises therapy with a vinca alkaloid or analog. Examples of vinca alkaloids and analogs include vinblastine, vincristine, vindesine, vinorelbine, vinflunine. In some cases, the anticancer therapy comprises therapy with a podophyllotoxin derivative. Examples of podophyllotoxin derivates include etoposide, teniposide. In certain cases, the anticancer therapy comprises therapy with a colchicine derivative. Examples of colchicine derivates include demecolcine. In some cases, the anticancer therapy comprises therapy with a taxane. Examples of taxanes include paclitaxel, docetaxel, paclitaxel poliglumex. In certain cases, the anticancer therapy comprises therapy with other plant alkaloids and natural products. Examples of other plant alkaloids and natural products include trabectedin.

In some cases, the anticancer therapy comprises therapy with chemotherapeutic cytotoxic antibiotics and related substances. Examples of cytotoxic antibiotics and related substances include actinomycines, anthracyclines and related substances, and other cytotoxic antibiotics.

In certain cases, the anticancer therapy comprises therapy with an actinomycin cytotoxic antibiotic. Examples of actinomycin cytotoxic antibiotics include dactinomycin. In some cases, the anticancer therapy comprises therapy with an anthracyclin cytotoxic antibiotic. Examples of anthracyclin cytotoxic antibiotics include doxorubicin, daunorubicin, epirubicin, aclarubicin, zorubicin, idarubicin, mitoxantrone, pirarubicin, valrubicin. In certain cases, the anticancer therapy comprises therapy with other cytotoxic antibiotics. Examples of other cytotoxic antibiotics include bleomycin, plicamycin, mitomycin, ixabepilone.

In some cases, the anticancer therapy comprises therapy with other antineoplastic agents. Examples of other antineoplastic agents include platinum compounds and methyl hydrazines.

In certain cases, the anticancer therapy comprises therapy with an antineoplastic platinum compound. Examples of antineoplastic platinum compounds include cisplatin, carboplatin, oxaliplatin, satraplatin. In some cases, the anticancer therapy comprises therapy with an antineoplastic methylhydrazine. Examples of antineoplastic methylhydrazines include procarbazine.

### Alkylating Agent Therapy

In certain cases, a pharmaceutical composition comprising an anticancer agent and two BER pathway inhibitors, such as methoxyamine and a PARP inhibitor, potentiates the cytotoxic activity of said anticancer agent. In some cases, a pharmaceutical composition comprising an alkylating anticancer agent and two BER pathway inhibitors, such as methoxyamine and a PARP inhibitor potentiates the cytotoxic activity of said alkylating anticancer agent. In certain cases, the two BER pathway inhibitors have a different mode of action. In some cases, a pharmaceutical composition comprising an alkylating anticancer agent, an AP site binder, and a PARP inhibitor potentiate the cytotoxic activity of said alkylating anticancer agent. In certain cases, a pharmaceutical composition comprising an alkylating anticancer agent, the AP site binder methoxyamine (MX), and a PARP inhibitor potentiate the cytotoxic activity of said alkylating anticancer agent. In some cases, a pharmaceutical composition comprising an alkylating anticancer agent, methoxyamine, and the PARP inhibitor ABT-888 potentiates the cytotoxic activity of said alkylating anticancer agent. In certain cases, the cytotoxic activity of the alkylating cytotoxic anticancer agent is synergistically potentiated by the two BER pathway inhibitors. In some cases, a pharmaceutical composition comprising an alkylating anticancer agent, an AP site binder, and a PARP inhibitor synergistically potentiates the cytotoxic activity of said alkylating anticancer agent. In certain cases, a pharmaceutical composition comprising an alkylating anticancer agent, the AP site binder methoxyamine (MX), and a PARP inhibitor synergistically potentiates the cytotoxic activity of said alkylating anticancer agent. In some cases, a pharmaceutical composition comprising an alkylating anticancer agent, the AP site binder methoxyamine (MX), and the PARP inhibitor ABT-888 synergistically potentiates the cytotoxic activity of said alkylating anticancer agent.

In certain cases, the alkylating anticancer agent is selected from, but not limited to, cyclophosphamide, chlorambucil, melphalan, chlormethine, ifosfamide, trofosfamide, prednimustine, bendamustine, busulfan, treosulfan, mannosulfan, thiotepa, triaziquone, carboquone, carmustine, lomustine, semustine, streptozocin, fotemustine, nimustine, ranimustine, etoglucid, mitobronitol, pipbroman, temozolomide (TMZ), dacarbazine.

In some cases, a pharmaceutical composition comprising temozolomide (TMZ) and two BER pathway inhibitors, such as methoxyamine and a PARP inhibitor, potentiates the cytotoxic activity of said TMZ. In certain cases, a pharmaceutical composition comprising TMZ, methoxyamine, and a PARP inhibitor potentiate the cytotoxic activity of said TMZ. In some cases, a pharmaceutical composition comprising TMZ, methoxyamine, and the PARP inhibitor ABT-888 potentiates the cytotoxic activity of said TMZ. In some cases, a pharmaceutical composition comprising TMZ, methoxyamine, and a PARP inhibitor synergistically potentiates the cytotoxic activity of said TMZ. In some cases, a pharmaceutical composition comprising TMZ, methoxyamine, and the PARP inhibitor ABT-888 synergistically potentiates the cytotoxic activity of said TMZ. In certain instances, synergistically means that the resulting potentiation of the cytotoxicity of anticancer therapy is greater than just adding the potentiating effect that the individual BER pathway inhibitor (such as methoxyamine or a PARP inhibitor) has on anticancer therapy. In some instances, synergistically also means that there is significant potentiation of the cytotoxic activity of the anticancer agent in combination with two BER pathway inhibitors (such as methoxyamine and a PARP inhibitor), when there is no significant potentiation of the cytotoxic activity of the anticancer agent in combination with just one BER pathway inhibitor individually.

### Radiation Therapy

Radiation therapy also works by damaging the DNA of cells. The damage is caused by a photon, electron, proton, neutron, or ion beam directly or indirectly ionizing the atoms which make up the DNA chain. Indirect ionization happens as a result of the ionization of water, forming free radicals, notably hydroxyl radicals, which then damage the DNA. In the most common forms of radiation therapy, most of the radiation effect is through free radicals. Because cells have mechanisms for repairing DNA damage, breaking the DNA on both strands proves to be the most significant technique in modifying cell characteristics.

Tumor cells have shown increased sensitivity to gamma- and X-radiation in the presence of PARP inhibitors. Radiosensitization by PARP inhibition seems to have greater effect on cells in the S and G2 phases of the cell cycle, and non-cycling cells exhibit minimal sensitivity. Although radiosensitization is partly due to the inhibition of SSB repair, it is likely that double stranded DNA breaks (DSB) repair, which is more cytotoxic, is also affected.

In some cases, a pharmaceutical composition comprising two BER pathway inhibitors, such as methoxyamine and a PARP inhibitor, potentiates the cytotoxic activity of radiation therapy. In certain cases, the two BER pathway inhibitors have a different mode of action. In some cases, the two BER pathway inhibitors are a PARP inhibitor and an AP site binder. In certain cases, a pharmaceutical composition comprising an AP site binder and a PARP inhibitor potentiate the cytotoxic activity of radiation therapy. In some cases, a pharmaceutical composition comprising the AP site binder methoxyamine (MX) and a PARP inhibitor potentiates the cytotoxic activity of radiation therapy. In certain cases, a pharmaceutical composition comprising methoxyamine and the PARP inhibitor ABT-888 potentiates the cytotoxic activity of radiation therapy. In some cases, the cytotoxic activity of radiation therapy is synergistically potentiated by the two BER pathway inhibitors, such as methoxyamine and a PARP inhibitor. In certain cases, a pharmaceutical composition comprising methoxyamine and a PARP inhibitor synergistically potentiates the cytotoxic activity of radiation therapy. In some cases, a pharmaceutical composition comprising methoxyamine and the PARP inhibitor ABT-888 synergistically potentiates the cytotoxic activity of radiation therapy. In certain instances, synergistically means that the resulting potentiation of the cytotoxicity activity of radiation therapy is greater than just adding the potentiating effect that the individual BER pathway inhibitor (such as methoxyamine or a PARP inhibitor) has on radiation therapy. In some instances, synergistically also means that there is significant potentiation of the cytotoxic activity of radiation therapy in combination with two BER pathway inhibitors (such as methoxyamine or a PARP inhibitor), when there is no significant potentiation of the cytotoxic activity of radiation therapy in combination with just one BER pathway inhibitor individually.

### Antimetabolite Therapy

Antimetabolites are used in cancer therapy to disrupt RNA and DNA production and induce cell death. Many DNA lesions created by antimetabolite chemotherapy are repaired by BER.

Inducible DNA repair is required by cells to counteract the harmful effects of continuous exposure to environmental and endogenous DNA damaging agents. Base excision repair (BER) is responsible for handling a diverse array of DNA lesions arising as a result of intrinsic DNA instability or reactive species of both endogenous and exogenous origin. In BER, the repair of all lesions is funneled through the glycosylases-mediated generation of apurinic/apyrimidic (AP) sites which are resolved as a result of downstream pathway activity. The BER pathway is comprised of three major steps: damage recognition and base excision by a damage specific DNA glycosylase; phosphodiester bond cleavage and generation of a single strand break by AP endonuclease (APE); nucleotide addition by DNA polymerase and gap ligation by DNA ligases. In some instances, induction of various DNA glycosylases is one component of the cellular response to DNA damage. In certain instances, glycosylase induction enhances lesion repair through increased excision of modified bases. However, in some instances, increased glycosylase expression also results in AP site accumulation which can lead to double strand breaks (DSBs) or random base incorporation during semi conservative replication. Uracil DNA glycosylase (UDG) is the major DNA glycosylase for the removal of uracil, arising from incorporation of uracil during replication or spontaneous deamination of cytosine throughout the genome.

UDG mRNA and protein is enhanced in response to treatment with antimetabolites, like e.g., fludarabine and pemetrexed. This UDG induction is coupled with DNA strand breaks and apoptotic signaling. Methoxyamine (MX) blockage of BER further exacerbates the DNA damage response and potentiates cytotoxicity of antimetabolites and also potentiates UDG expression. UDG induction contributes to increased AP site formation and MX-bound AP sites. These lesions act as toposiomerase II alpha (topo II) substrates, leading to topo II-mediated double strand breaks (DSBs) and apoptosis.

In specific instances, the antifolate antimetabolite pemetrexed inhibits thymidylate synthetase (TS) which causes a reduction of dTTP levels with a concomitant rise in dUTP. In this milieu, uracil is aberrantly incorporated into the genome, removed by uracil DNA glycosylase (UDG), and reincorporated during DNA replication. In some instances, MX inhibits BER by binding and stabilizing abasic (AP) sites after glycosylase removal of abnormal bases and potentiates the cytotoxicity of pemetrexed in lung, breast, and colon cancer cells. In certain instances, MX enhances the cytotoxicity of the antimetabolite fludarabine, a purine analog, in human leukemia (Jurkat) cells, and decitabine in colon cancer, melanoma and primary acute myelogenous leukemia cells.

In some cases, a pharmaceutical composition comprising an antimetabolite anticancer agent and two BER pathway inhibitors, such as methoxyamine and a PARP inhibitor, potentiates the cytotoxic activity of said antimetabolite anticancer agent. In some cases, a pharmaceutical composition comprising an antimetabolite anticancer agent, an AP site binder, and a PARP inhibitor potentiate the cytotoxic activity of said antimetabolite anticancer agent. In certain cases, a pharmaceutical composition comprising an antimetabolite anticancer agent, the AP site binder methoxyamine, and a PARP inhibitor potentiate the cytotoxic activity of said antimetabolite anticancer agent. In some cases, a pharmaceutical composition comprising an antimetabolite anticancer agent, methoxyamine, and the PARP inhibitor ABT-888 potentiates the cytotoxic activity of said antimetabolite anticancer agent. In certain cases, the cytotoxic activity of the antimetabolite cytotoxic anticancer agent is synergistically potentiated by the two BER pathway inhibitors, such as methoxyamine and a PARP inhibitor. In some cases, a pharmaceutical composition comprising an antimetabolite anticancer agent, methoxyamine, and a PARP inhibitor synergistically potentiates the cytotoxic activity of said antimetabolite anticancer agent. In some cases, a pharmaceutical composition comprising an antimetabolite anticancer agent, methoxyamine, and the PARP inhibitor ABT-888 synergistically potentiates the cytotoxic activity of said antimetabolite anticancer agent.

In certain cases, the antimetabolite anticancer agent is selected from, but not limited to, methotrexate, ralitrexed, pemetrexed, pralatrexate, mercaptopurine, thioguanine, clabridine, fludarabine, clofarabine, nelarabine, cytarabine, fluorouracil, tegafur, carmofur, gemcitabine, capecitabine, azacitidine, decitabine, fluorouracil combinations, and tegafur combinations.

In some cases, a pharmaceutical composition comprising pemetrexed and two BER pathway inhibitors potentiates the cytotoxic activity of said pemetrexed. In certain cases, a pharmaceutical composition comprising pemetrexed, methoxyamine, and a PARP inhibitor potentiate the cytotoxic activity of said pemetrexed. In some cases, a pharmaceutical composition comprising pemetrexed, methoxyamine, and the PARP inhibitor ABT-888 potentiates the cytotoxic activity of said pemetrexed. In certain cases, the cytotoxic activity of pemetrexed is synergistically potentiated by the two BER pathway inhibitors. In some cases, a pharmaceutical composition comprising pemetrexed, methoxyamine, and a PARP inhibitor synergistically potentiates the cytotoxic activity of said pemetrexed. In some cases, a pharmaceutical composition comprising pemetrexed, methoxyamine, and the PARP inhibitor ABT-888 synergistically potentiates the cytotoxic activity of said pemetrexed. In certain instances, synergistically means that the resulting potentiation of the cytotoxicity of anticancer therapy is greater than just adding the potentiating effect that the individual BER pathway inhibitor (such as methoxyamine or a PARP inhibitor) has on anticancer therapy. In some instances, synergistically also means that there is significant potentiation of the cytotoxic activity of the anticancer agent in combination with two BER pathway inhibitors (such as methoxyamine and a PARP inhibitor), when there is no significant potentiation of the cytotoxic activity of the anticancer agent in combination with just one BER pathway inhibitor individually.

### Methods of Treatment (not part of the invention)

In some cases, described herein is a method of treating cancer, said method comprising administering to an individual in need thereof two BER pathway inhibitors (such as methoxyamine and a PARP inhibitor), and an anticancer agent, wherein the two BER pathway inhibitors potentiate the cytotoxic activity of the anticancer agent.

In certain cases, described herein is a method of treating cancer, said method comprising administering to an individual in need thereof two BER pathway inhibitors (such as methoxyamine and a PARP inhibitor) and an alkylating anticancer agent, wherein the two BER pathway inhibitors potentiate the cytotoxic activity of said alkylating anticancer agent.

In some cases, the method of treating cancer comprises administering an alkylating agent anticancer agent, an AP site binder, and a PARP inhibitor to an individual in need thereof, wherein the cytotoxic activity of said alkylating anticancer agent is potentiated. In certain cases, the method of treating cancer comprises administering an alkylating anticancer agent, methoxyamine, and a PARP inhibitor, wherein the cytotoxic activity of said alkylating anticancer agent is potentiated. In some cases, the method of treating cancer comprises administering an alkylating anticancer agent, methoxyamine, and the PARP inhibitor ABT-888, wherein the cytotoxic activity of said alkylating anticancer agent is potentiated. In certain cases, the cytotoxic activity of the alkylating cytotoxic anticancer agent is synergistically potentiated by the two BER pathway inhibitors, such as methoxyamine and a PARP inhibitor. In certain cases, the method of treating cancer comprises administering an alkylating anticancer agent, methoxyamine, and a PARP inhibitor, wherein the cytotoxic activity of said alkylating anticancer agent is synergistically potentiated. In some cases, the method of treating cancer comprises administering an alkylating anticancer agent, methoxyamine, and the PARP inhibitor ABT-888, wherein the cytotoxic activity of said alkylating anticancer agent is synergistically potentiated.

In certain cases, the alkylating anticancer agent is selected from, but not limited to, cyclophosphamide, chlorambucil, melphalan, chlormethine, ifosfamide, trofosfamide, prednimustine, bendamustine, busulfan, treosulfan, mannosulfan, thiotepa, triaziquone, carboquone, carmustine, lomustine, semustine, streptozocin, fotemustine, nimustine, ranimustine, etoglucid, mitobronitol, pipbroman, temozolomide (TMZ), dacarbazine.

In some cases, a method of treating cancer comprising administering temozolomide (TMZ) and two BER pathway inhibitors (such as methoxyamine and a PARP inhibitor) potentiates the cytotoxic activity of said TMZ. In certain cases, the two BER pathway inhibitors have a different mode of action. In some cases, the method of treating cancer comprises administering temozolomide (TMZ), an AP site binder, and a PARP inhibitor to an individual in need thereof, wherein the cytotoxic activity of said TMZ is potentiated. In certain cases, the method of treating cancer comprises administering TMZ, the AP site binder methoxyamine (MX), and a PARP inhibitor, wherein the cytotoxic activity of said TMZ is potentiated. In some cases, the method of treating cancer comprises administering TMZ, methoxyamine, and the PARP inhibitor ABT-888, wherein the cytotoxic activity of said TMZ is potentiated. In certain cases, the cytotoxic activity of TMZ is synergistically potentiated by the two BER pathway inhibitors, such as methoxyamine and a PARP inhibitor. In certain cases, the method of treating cancer comprises administering TMZ, the AP site binder methoxyamine (MX), and a PARP inhibitor, wherein the cytotoxic activity of said TMZ is synergistically potentiated. In some cases, the method of treating cancer comprises administering TMZ, methoxyamine, and the PARP inhibitor ABT-888, wherein the cytotoxic activity of said TMZ is synergistically potentiated. In certain instances, synergistically means that the resulting potentiation of the cytotoxicity of anticancer therapy is greater than just adding the potentiating effect that the individual BER pathway inhibitor (such as methoxyamine or a PARP inhibitor) has on anticancer therapy. In some instances, synergistically also means that there is significant potentiation of the cytotoxic activity of the anticancer agent in combination with two BER pathway inhibitors (such as methoxyamine and a PARP inhibitor), when there is no significant potentiation of the cytotoxic activity of the anticancer agent in combination with just one BER pathway inhibitor individually.

In certain cases, described herein is a method of treating cancer, said method comprising administering to an individual in need thereof two BER pathway inhibitors (such as methoxyamine and a PARP inhibitor) and an antimetabolite anticancer agent, wherein the two BER pathway inhibitors potentiate the cytotoxic activity of said antimetabolite anticancer agent. In some cases, the two BER pathway inhibitors have a different mode of action.

In some cases, the method of treating cancer comprises administering an antimetabolite agent anticancer agent, an AP site binder, and a PARP inhibitor to an individual in need thereof, wherein the cytotoxic activity of said antimetabolite anticancer agent is potentiated. In certain cases, the method of treating cancer comprises administering an antimetabolite anticancer agent, methoxyamine, and a PARP inhibitor, wherein the cytotoxic activity of said antimetabolite anticancer agent is potentiated. In some cases, the method of treating cancer comprises administering an antimetabolite anticancer agent, methoxyamine, and the PARP inhibitor ABT-888, wherein the cytotoxic activity of said antimetabolite anticancer agent is potentiated. In certain cases, the cytotoxic activity of the antimetabolite anticancer agent is synergistically potentiated by the two BER pathway inhibitors, such as methoxyamine and a PARP inhibitor. In certain cases, the method of treating cancer comprises administering an antimetabolite anticancer agent, methoxyamine, and a PARP inhibitor, wherein the cytotoxic activity of said antimetabolite anticancer agent is synergistically potentiated. In some cases, the method of treating cancer comprises administering an antimetabolite anticancer agent, methoxyamine, and the PARP inhibitor ABT-888, wherein the cytotoxic activity of said antimetabolite anticancer agent is synergistically potentiated.

In certain cases, the antimetabolite anticancer agent is selected from, but not limited to, methotrexate, ralitrexed, pemetrexed, pralatrexate, mercaptopurine, thioguanine, clabridine, fludarabine, clofarabine, nelarabine, cytarabine, fluorouracil, tegafur, carmofur, gemcitabine, capecitabine, azacitidine, decitabine, fluorouracil combinations, and tegafur combinations.

In certain cases, the method of treating cancer comprises administering pemetrexed, methoxyamine, and a PARP inhibitor, wherein the cytotoxic activity of said pemetrexed is potentiated. In some cases, the method of treating cancer comprises administering pemetrexed, methoxyamine, and the PARP inhibitor ABT-888, wherein the cytotoxic activity of said pemetrexed is potentiated. In certain cases, the cytotoxic activity of the pemetrexed is synergistically potentiated by the two BER pathway inhibitors, such a methoxyamine and a PARP inhibitor. In certain cases, the method of treating cancer comprises administering pemetrexed, methoxyamine, and a PARP inhibitor, wherein the cytotoxic activity of said pemetrexed is synergistically potentiated. In some cases, the method of treating cancer comprises administering pemetrexed, methoxyamine, and the PARP inhibitor ABT-888, wherein the cytotoxic activity of said pemetrexed is synergistically potentiated.

In some cases, described herein is a method of treating cancer, comprising administering to an individual in need thereof radiation therapy and two BER pathway inhibitors (such as methoxyamine and a PARP inhibitor), wherein the two BER pathway inhibitors potentiate the effectiveness of radiation therapy. In certain cases, the two BER pathway inhibitors have a different mode of action. In some cases, the two BER pathway inhibitors are a PARP inhibitor and an AP site binder. In some cases, the method of treating cancer comprises radiation therapy, methoxyamine, and a PARP inhibitor, wherein the effectiveness of radiation therapy is potentiated. In certain cases, the method of treating cancer comprises radiation therapy, methoxyamine, and the PARP inhibitor ABT-888, wherein the effectiveness of radiation therapy is potentiated. In some cases, the cytotoxic activity of radiation therapy is synergistically potentiated by the two BER pathway inhibitors, such as methoxyamine and a PARP inhibitor. In some cases, the method of treating cancer comprises radiation therapy, methoxyamine, and a PARP inhibitor, wherein the effectiveness of radiation therapy is synergistically potentiated. In certain cases, the method of treating cancer comprises radiation therapy, methoxyamine, and the PARP inhibitor ABT-888, wherein the effectiveness of radiation therapy is synergistically potentiated.

In some cases, described herein is a method of treating cancer, said method comprising administering to an individual in need thereof two BER pathway inhibitors (such as methoxyamine and a PAPR inhibitor) in combination with an anticancer agent or radiation therapy, wherein the two BER pathway inhibitors potentiate the cytotoxic activity of the anticancer agent or radiation therapy.

In certain cases, the cancer is susceptible to cytotoxic anticancer therapy with either radiation therapy or anticancer agents. In some cases the cancer is acute lymphoblastic leukemia, acute myeloid leukemia, adrenocortical carcinoma, anal cancer, appendix cancer, astrocytomas, basal cell carcinoma, bile duct cancer, bladder cancer, bone cancer, brain tumor, breast cancer, bronchial tumors, Burkitt lymphoma, CNS lymphoma, cervical cancer, chordoma, chronic lymphocytic leukemia, chronic myelogenous leukemia, colon cancer, colorectal cancer, endometrial cancer, esophageal cancer, Ewing sarcoma, eye cancer, gallbladder cancer, gastric cancer, gastrointestinal carcinoid tumor, gastrointestinal stromal cancer, germ cell tumors, gestational trophoblastic tumor, glioma, hairy cell leukemia, head and neck cancer, hepatocellular cancer, Hodgkin lymphoma, hypopharyngeal cancer, intraocular melanoma, islet cell tumor, Kaposi sarcoma, kidney cancer, Langerhans cell histiocytosis, laryngeal cancer, lip and oral cavity cancer, liver cancer, lung cancer (non-small cell), lung cancer (small cell), medulloblastoma, medulloepithelioma, melanoma, Merkel cell carcinoma, mesothelioma, metastatic squamous neck cancer, mouth cancer, multiple myeloma, mycosis fungoides, myelodysplastic syndromes, nasal cavity and paranasal sinus cancer, nasopharyngeal cancer, neuroblastoma, non-Hodgkin lymphoma, oropharyngeal cancer, osteosarcoma, ovarian cancer, pancreatic cancer, polillomatosis, parathyroid cancer, pharyngeal cancer, pineoplastoma, pituary tumor, pleuropulmonary blastoma, prostate cancer, rectal cancer, renal cell cancer, retinoblastoma, rhabdomyosarcoma, salivary gland cancer, skin cancer (non-melanoma), small intestine cancer, soft tissue sarcoma, squamous cell carcinoma, stomach cancer, T-cell lymphoma, testicular cancer, throat cancer, thymonoma, thyroid cancer, urethral cancer, uterine sarcoma, vaginal cancer, vulvar cancer, Waldenstrom macroglobulinemia, Wilms tumor.

In certain cases, the cancer is bladder cancer, brain cancer, breast cancer, cervical cancer, colon and rectal cancer, glioblastoma multiform, hepatocellular cancer, kidney (renal) cancer, leukemia, lung cancer, non-small-cell lung cancer, melanoma, mesothelioma, non-Hodgkin lymphoma, ovarian cancer, pancreatic cancer, prostate cancer, skin cancer (non-melanoma), thyroid cancer.

A tumor or cancer to be treated in the methods described herein includes, but is not limited to, a lung cancer, a gynecologic malignancy, a melanoma, a breast cancer, a brain cancer (e.g., glioblastoma multiforme, "GBM") a pancreatic cancer, an ovarian cancer, a uterine cancer, a colorectal cancer, a prostate cancer, a kidney cancer, a head cancer, a liver cancer (hepatocellular cancer), a uterine cancer, a neck cancer, a kidney cancer (renal cell cancer), a sarcoma, a myeloma, and lymphoma. In one case, a tumor to be treated is a solid or semi-solid tumor. In another case, a tumor to be treated is a primary tumor. In another case, a tumor to be treated is a metastatic tumor. In one case, a tumor or cancer to be treated is of epithelial origin. In another case, the cancer to be treated is myeloma. In another case, the cancer to be treated is ovarian cancer. In another case, the cancer to be treated is kidney/renal cancer. In yet another case, the cancer to be treated is hepatocellular/liver cancer.

### Lung cancer

In one aspect, provided herein is a method to treat lung cancer. The most common type of lung cancer is non-small cell lung cancer (NSCLC), which accounts for approximately 80-85% of lung cancers and is divided into squamous cell carcinomas, adenocarcinomas, and large cell undifferentiated carcinomas. Small cell lung cancer accounts for 15-20% of lung cancers.

Lung cancer staging is an assessment of the degree of spread of the cancer from its original source. It is an important factor affecting the prognosis and potential treatment of lung cancer. Non-small cell lung carcinoma is staged from IA ("one A"; best prognosis) to IV ("four"; worst prognosis). Small cell lung carcinoma is classified as limited stage if it is confined to one half of the chest and within the scope of a single radiotherapy field; otherwise, it is extensive stage.

Non-small cell lung cancer may be staged using EUS (endoscopic ultrasound) or CT or MRI scan or at surgery to classify the extent of disease according to the TNM system. These subjects undergo staging as part of the process of considering prognosis and treatment. The AJCC recommends TNM staging followed by further grouping.

Primary tumor (T): TX: The primary tumor cannot be assessed, or there are malignant cells in the sputum or bronchoalveolar lavage but not seen on imaging or bronchoscopy; Tis: Carcinoma in situ. T0: No evidence of primary tumor. T1: Tumor less than 3 cm in its greatest dimension, surrounded by lung or visceral pleura and without bronchoscopic invasion into the main bronchus. T2: A tumor with any of: more than 3 cm in greatest dimension; extending into the main bronchus (but more than 2 cm distal to the carina), and obstructive pneumonitis (but not involving the entire lung). T3: A tumor with any of: invasion of the chest wall, diaphragm, mediastinal pleura, or parietal pericardium; extending into the main bronchus, within 2 cm of the carina, but not involving the carina; and obstructive pneumonitis of the entire lung. T4: A tumor with any of: invasion of the mediastinum, heart, great vessels, trachea, esophagus, vertebra, or carina; separate tumor nodules in the same lobe; and malignant pleural effusion. Lymph nodes (N): NX: Lymph nodes cannot be assessed; N0: No lymph nodes involved; N1: Metastasis to ipsilateral peribronchial or ipsilateral hilar lymph nodes; N2: Metastasis to ipsilateral mediastinal or subcarinal lymph nodes; and N3: Metastasis to any of: ipsilateral supraclavicular lymph nodes; ipsilateral scalene lymph nodes; and contralateral lymph nodes. Distant metastasis (M): MX: Distant metastasis cannot be assessed; M0: No distant metastasis; and M1: Distant metastasis is present.

### Uterine cancers / Gynecologic Malignancy

Uterine cancers may refer to any of several different types of cancer which occur in the uterus, namely: uterine sarcomas (e.g., sarcomas of the myometrium, or muscular layer of the uterus, are most commonly leiomyosarcomas); endometrial cancer; and cervical cancer.

In another aspect, provided herein is a method to treat endometrium cancer. Endometrial cancer is a cancer that starts in the endometrium, the inner lining of the uterus. Some of the examples of the cancer of uterus and endometrium include, but are not limited to, adenocarcinomas, adenoacanthomas, adenosquamous carcinomas, papillary serous adenocarcinomas, clear cell adenocarcinomas, uterine sarcomas, stromal sarcomas, malignant mixed mesodermal tumors, and leiomyosarcomas.

In another aspect, the method treats cervical cancer, preferably an adenocarcinoma in the cervix epithelial. Two main types of this cancer exist: squamous cell carcinoma and adenocarcinomas. The former constitutes about 80-90% of all cervical cancers and develops where the ectocervix (portion closest to the vagina) and the endocervix (portion closest to the uterus) join. The latter develop in the mucous-producing gland cells of the endocervix. Some cervical cancers have characteristics of both of these and are called adenosquamous carcinomas or mixed carcinomas.

### Ovarian cancer

In another aspect, provided herein is a method of treating ovarian cancer, including epithelial ovarian tumors.

Ovarian cancer is classified according to the histology of the tumor, obtained in a pathology report. Surface epithelial-stromal tumor, also known as ovarian epithelial carcinoma, is the most typical type of ovarian cancer. It includes serous tumor, endometrioid tumor and mucinous cystadenocarcinoma. Sex cord-stromal tumor, including estrogen-producing granulosa cell tumor and virilizing Sertoli-Leydig cell tumor or arrhenoblastoma, accounts for 8% of ovarian cancers. Germ cell tumor accounts for approximately 30% of ovarian tumors but only 5% of ovarian cancers because most germ cell tumors are teratomas and most teratomas are benign. Germ cell tumor tends to occur in young women and girls. The prognosis depends on the specific histology of germ cell tumor, but overall is favorable. Mixed tumors contain elements of more than one of the above classes of tumor histology.

Ovarian cancer can also be a secondary cancer, the result of metastasis from a primary cancer elsewhere in the body. Common primary cancers are breast cancer and gastrointestinal cancer (in which case the ovarian cancer is a Krukenberg cancer). Surface epithelial-stromal tumor can originate in the peritoneum (the lining of the abdominal cavity), in which case the ovarian cancer is secondary to primary peritoneal cancer, but treatment is basically the same as for primary surface epithelial-stromal tumor involving the peritoneum.

Ovarian cancer staging is by the FIGO staging system and uses information obtained after surgery, which can include a total abdominal hysterectomy, removal of both ovaries and fallopian tubes, the omentum, and pelvic (peritoneal) washings for cytology. The AJCC stage is the same as the FIGO stage.

Stage I refers to ovarian cancer limited to one or both ovaries: IA - involves one ovary; capsule intact; no tumor on ovarian surface; no malignant cells in ascites or peritoneal washings; IB - involves both ovaries; capsule intact; no tumor on ovarian surface; negative washings; and IC - tumor limited to ovaries with any of the following: capsule ruptured, tumor on ovarian surface, positive washings.

Stage II refers to pelvic extension or implants: IIA - extension or implants onto uterus or fallopian tube; negative washings; IIB - extension or implants onto other pelvic structures; negative washings; and IIC - pelvic extension or implants with positive peritoneal washings

Stage III refers to microscopic peritoneal implants outside of the pelvis; or limited to the pelvis with extension to the small bowel or omentum: IIIA - microscopic peritoneal metastases beyond pelvis; IIIB - macroscopic peritoneal metastases beyond pelvis less than 2 cm in size; and IIIC - peritoneal metastases beyond pelvis > 2 cm or lymph node metastases

Stage IV refers to distant metastases to the liver or outside the peritoneal cavity.

Para-aortic lymph node metastases are considered regional lymph nodes (Stage IIIC).

In some cases, the methods described herein treat an ovarian cancer selected from the following: an adenocarcinoma in the ovary and an adenocarcinoma that has migrated from the ovary into the abdominal cavity.

### Melanoma

A melanoma is a malignant tumor of melanocytes which are found predominantly in skin but also in the bowel and the eye (uveal melanoma). It is one of the rarer types of skin cancer but causes the majority of skin cancer related deaths. Malignant melanoma is a serious type of skin cancer caused by uncontrolled growth of pigment cells, called melanocytes. Melanomas also include, but are not limited to, a choroidea melanoma, malignant melanomas, cutaneous melanomas and intraocular melanomas.

Melanoma may be divided into the following types: Lentigo maligna, Lentigo maligna melanoma, superficially spreading melanoma, acral lentiginous melanoma, mucosal melanoma, nodular melanoma, polypoid melanoma, desmoplastic melanoma, amelanotic melanoma, soft-tissue melanoma, and uveal melanoma. Melanoma stages are as follows:
Stage 0 - melanoma in situ (Clark Level I).
Stage I/II - invasive melanoma: T1a: less than 1.00 mm primary, without ulceration, Clark Level II-III; T1b: less than 1.00 mm primary, with ulceration or Clark Level IV-V; and T2a: 1.00-2.00 mm primary, without ulceration.
Stage II - High Risk Melanoma: T2b: 1.00-2.00 mm primary, with ulceration; T3a: 2.00-4.00 mm primary, without ulceration; T3b: 2.00-4.00 mm primary, with ulceration; T4a: 4.00 mm or greater primary without ulceration; and T4b: 4.00 mm or greater primary with ulceration.
Stage III - Regional Metastasis: N1: single positive lymph node; N2: 2-3 positive lymph nodes or regional skin/in-transit metastasis; and N3: 4 positive lymph nodes or lymph node and regional skin/in transit metastases.
Stage IV - Distant Metastasis: M1a: Distant Skin Metastasis, Normal LDH; M1b: Lung Metastasis, Normal LDH; and M1c: Other Distant Metastasis OR Any Distant Metastasis with Elevated LDH.
In one case, the methods described herein treat a melanoma.

### Colon Cancer and Colorectal Cancer

Colorectal cancer (also called colon cancer or large bowel cancer) includes cancerous growths in the colon, rectum (anus) and appendix. With 655,000 deaths worldwide per year, it is the third most common form of cancer and the second leading cause of cancer-related death in the Western world. Many colorectal cancers are thought to arise from adenomatous polyps in the colon. These mushroom-like growths are usually benign, but some may develop into cancer over time.

In another case, Dukes classification may be used to classify colorectal cancer based on stages A-D. Stage A refers to colorectal cancer that is limited to mucosa (i.e., has not invaded through the bowel wall). Stage B1 refers to extending into muscularis propria, but not penetrating through it (i.e., lymph nodes have not been invaded); whereas Stage B2 cancer has penetrated through the muscularis propria, but not penetrating through it (i.e., lymph nodes have not been invaded). Stage C1 refers to cancer that extends into the muscularis propria, but not penetrating through it (i.e., lymph nodes are involved); whereas Stage C2 refers to cancer that extends into the muscularis propria and penetrating through it (i.e., lymph nodes are involved). Stage D refers to distant metastatic spread. The TNM system may also be used to stage colorectal cancer according to conventional means known in the art.

### Breast cancer

Several types of breast cancer exist that may be treated by the methods described herein. A lobular carcinoma *in situ* and a ductal carcinoma *in situ* are breast cancers that have developed in the lobules and ducts, respectively, but have not spread to the fatty tissue surrounding the breast or to other areas of the body. Infiltrating (or invasive) lobular and ductal carcinoma are cancers that have developed in the lobules and ducts, respectively, and have spread to either the breast's fatty tissue and/or other parts of the body. In one aspect, provided herein is a method of treating breast cancer, such as a ductal carcinoma in duct tissue in a mammary gland, a breast cancer that is Her2-and/or ER- and/or PR-.Other cancers of the breast that would benefit from treatment by the methods are medullary carcinomas, colloid carcinomas, tubular carcinomas, and inflammatory breast cancer.

In one case, breast cancer is staged according to the TNM system. Prognosis is closely linked to results of staging, and staging is also used to allocate patients to treatments both in clinical trials and clinical practice.

Briefly, the information for staging is as follows: TX: Primary tumor cannot be assessed. T0: No evidence of tumor. Tis: Carcinoma in situ, no invasion; T1: Tumor is 2 cm or less; T2: Tumor is more than 2 cm but not more than 5 cm; T3: Tumor is more than 5 cm; T4: Tumor of any size growing into the chest wall or skin, or inflammatory breast cancer. NX: Nearby lymph nodes cannot be assessed N0: cancer has not spread to regional lymph nodes. N1: cancer has spread to 1 to 3 maxillary or one internal mammary lymph node N2: cancer has spread to 4 to 9 maxillary lymph nodes or multiple internal mammary lymph nodes N3: One of the following applies: cancer has spread to 10 or more maxillary lymph nodes, or cancer has spread to the lymph nodes under the clavicle (collar bone), or cancer has spread to the lymph nodes above the clavicle, or cancer involves maxillary lymph nodes and has enlarged the internal mammary lymph nodes, or cancer involves 4 or more maxillary lymph nodes, and tiny amounts of cancer are found in internal mammary lymph nodes on sentinel lymph node biopsy. MX: presence of distant spread (metastasis) cannot be assessed. M0: no distant spread. M1: spread to distant organs (not including the supraclavicular lymph node) has occurred.

### Pancreatic cancer

In another aspect, provided herein is a method of treating pancreatic cancer selected from the following: an epitheliod carcinoma in the pancreatic duct tissue and an adenocarcinoma in a pancreatic duct. The most common type of pancreatic cancer is an adenocarcinoma, which occurs in the lining of the pancreatic duct.

In one case, the methods described herein treat a pancreatic cancer.

### Prostate Cancer

In one other aspect, provided herein is a method to treat prostate cancer selected from the following: an adenocarcinoma or an adenocarcinoma that has migrated to the bone. Prostate cancer develops in the prostate organ in men, which surrounds the first part of the urethra. The prostate has several cell types but 99% of tumors are adenocarcinomas that develop in the glandular cells responsible for generating seminal fluid.

There are two schemes commonly used to stage prostate cancer. The most common is the TNM system, which evaluates the size of the tumor, the extent of involved lymph nodes, and any metastasis (distant spread). As with many other cancers, these are often grouped into four stages (I-IV). Another scheme, used less commonly, is the Whitmore-Jewett stage.

Briefly, Stage I disease is cancer that is found incidentally in a small part of the sample when prostate tissue was removed for other reasons, such as benign prostatic hypertrophy, and the cells closely resemble normal cells and the gland feels normal to the examining finger. In Stage II more of the prostate is involved and a lump can be felt within the gland. In Stage III, the tumor has spread through the prostatic capsule and the lump can be felt on the surface of the gland. In Stage IV disease, the tumor has invaded nearby structures, or has spread to lymph nodes or other organs. Grading is based on cellular content and tissue architecture from biopsies (Gleason) which provides an estimate of the destructive potential and ultimate prognosis of the disease.

In one case, the methods described herein treat a prostate cancer.

### Head and Neck Cancers

Head and neck cancers (e.g., oral, laryngeal, nasopharyngeal, esophageal, etc.), refer to a group of biologically similar cancers originating from the upper aerodigestive tract, including the lip, oral cavity (mouth), nasal cavity, paranasal sinuses, pharynx, and larynx. Most head and neck cancers are squamous cell carcinomas, originating from the mucosal lining (epithelium) of these regions. Head and neck cancers often spread to the lymph nodes of the neck, and this is often the first (and sometimes only) manifestation of the disease at the time of diagnosis. Head and neck cancer is strongly associated with certain environmental and lifestyle risk factors, including tobacco smoking, alcohol consumption, and certain strains of the sexually transmitted human papillomavirus. Management of patients with head and neck cancers remains a formidable task. Cancers such as, hypopharyngeal cancer, laryngeal cancer, nasopharyngeal cancer, oropharyngeal cancer, may be treated using the compounds described herein.

In one case, the methods described herein treat a head or neck cancer.

### Kidney cancer

In another aspect, provided herein is a method to treat kidney cancer. Kidney cancer (also called renal cell cancer, renal cell carcinoma, renal adenocarcinoma, and hypernephroma) is a disease in which malignant cells are found in the lining of tubules in the kidney. Renal cell carcinoma is the most common form of kidney cancer arising from the proximal renal tubule. It is the most common type of kidney cancer in adults, responsible for approximately 80% of cases.

In one case, the methods described herein treat a kidney cancer.

### Liver Cancer.

In another aspect, provided herein is a method to treat primary liver cancer (cancer that begins in the liver). Primary liver cancer can occur in both adults and children. Liver cancer is characterized by the presence of malignant hepatic tumors - tumors or growths on or in the liver. They may be discovered on medical imaging (even for a different reason than the cancer itself), or may be present in patients as an abdominal mass, abdominal pain, jaundice, or some other liver dysfunction. There are several types of liver cancer.

Hemangiomas: These are the most common type of benign liver tumor. They start in blood vessels. Most of these tumors do not cause symptoms, they do not need treatment. Some may bleed and need to be removed if it is mild to severe.

Hepatic adenomas: These benign epithelial liver tumors develop in the liver. They are, in most cases, located in the right hepatic lobe and are frequently seen as solitary. The size of adenomas range from 1 to 30 cm. Symptoms associated with hepatic adenomas are all associated with large lesions which can cause intense abdominal pain.

Focal nodular hyperplasia: Focal nodular hyperplasia (FNH) is the second most common tumor of the liver. This tumor is the result of a congenital arteriovenous malformation hepatocyte response. This process is one in which all normal constituents of the liver are present, but the pattern by which they are presented is abnormal. Even though those conditions exist the liver still seems to perform in the normal range.

Hepatocellular Cancer: Hepatocellular cancer (HCC) is the most common cancer of the liver. It is associated with alcohol abuse and hepatitis B infection and is particularly prevalent in Asia. The majority of HCC is detected at a time when cure by surgical resection is not possible; systemic treatment of un-resectable HCC is associated with survival of less than one year.

In one case, the methods described herein treat a liver cancer.

### Lymphoma

Lymphoma is a type of cancer that originates in lymphocytes of the immune system. They often originate in lymph nodes, presenting as an enlargement of the node (a tumor). Lymphomas are closely related to lymphoid leukemias, which also originate in lymphocytes but typically involve only circulating blood and the bone marrow (where blood cells are generated in a process termed haematopoesis) and do not usually form tumors. There are many types of lymphomas, and in turn, lymphomas are a part of the broad group of diseases called hematological neoplasms. Some forms of lymphoma are indolent (e.g. small lymphocytic lymphoma), compatible with a long life even without treatment, whereas other forms are aggressive (e.g. Burkitt's lymphoma), causing rapid deterioration and death.

The WHO Classification, published in 2001 and updated in 2008; http://en.wikipedia.org/wiki/Lymphoma - cite_note-isbn92-832-2411-6-2#cite_note-isbn92-832-2411-6-2 is the latest classification of lymphoma and is based upon the foundations laid within the "Revised European-American Lymphoma classification" (REAL). This system groups lymphomas by cell type (i.e., the normal cell type that most resembles the tumor) and defining phenotypic, molecular or cytogenetic characteristics. There are three large groups: the B cell, T cell, and natural killer cell tumors. Other less common groups are also recognized. Hodgkin's lymphoma, although considered separately within the WHO (and preceding) classifications, is now recognized as being a tumor of, albeit markedly abnormal, lymphocytes of mature B cell lineage.

In one case, the methods described herein treat a lymphoma.

### Sarcoma

A sarcoma is a cancer of the connective tissue (bone, cartilage, fat) resulting in mesoderm proliferation.

This is in contrast to carcinomas, which are of epithelial origin (breast, colon, pancreas, and others). However, due to an evolving understanding of tissue origin, the term "sarcoma" is sometimes applied to tumors now known to arise from epithelial tissue. The term soft tissue sarcoma is used to describe tumors of soft tissue, which includes elements that are in connective tissue, but not derived from it (such as muscles and blood vessels).

Sarcomas are given a number of different names, based on the type of tissue from which they arise. For example, osteosarcoma arises from bone, chondrosarcoma arises from cartilage, and leiomyosarcoma arises from smooth muscle. Sarcomas strike people in all age ranges, but they are very rare, accounting for only 1% of all cases of cancer. GIST is the most common form of sarcoma, with approximately 3000-3500 cases per year in the United States. This should be compared with breast cancer, with approximately 200,000 cases per year in North America.

Approximately 50% of bone sarcomas and 20% of soft tissue sarcomas are diagnosed in people under the age of 35. Some sarcomas, such as leiomyosarcoma, chondrosarcoma, and gastrointestinal stromal tumor (GIST), are more common in adults than in children. Most high grade bone sarcomas, including Ewing's sarcoma and osteosarcoma, are much more common in children and young adults.

In one case, the methods described herein treat a sarcoma.

### Carcinoma

A carcinoma is any malignant cancer that arises from epithelial cells. Carcinomas invade surrounding tissues and organs and may metastasize, or spread, to lymph nodes and other sites.

Carcinoma, like all neoplasia, is classified by its histopathological appearance. Adenocarcinoma and squamous cell carcinoma, two common descriptive terms for tumors, reflect the fact that these cells may have glandular or squamous cell appearances respectively. Severely anaplastic tumors might be so undifferentiated that they do not have a distinct histological appearance (undifferentiated carcinoma).

Sometimes a tumor is referred to by the presumptive organ of the primary (e.g., carcinoma of the prostate) or the putative cell of origin (hepatocellular carcinoma, renal cell carcinoma).

Adenocarcinoma is a malignant tumor originating in the epithelial cells of glandular tissue and forming glandular structures. This is common in the lung (forming 30-40% of all lung carcinomas). It is found peripherally, arising from goblet cells or type II pneumocytes.

Squamous cell carcinoma results from squamous metaplasia. This accounts for 20-30 percent of lung tumors and is usually hilar in origin.

Small cell carcinoma is almost certainly due to smoking. These metastasize early, and may secrete ADH (lowering patient sodium concentration).

Large cell undifferentiated carcinomas account for 10-15 percent of lung neoplasms. These are aggressive and difficult to recognize due to the undifferentiated nature. These are most commonly central in the lung.

Sinonasal undifferentiated carcinoma.

In one case, the methods described herein treat a carcinoma.

### Myeloma

Multiple myeloma (also known as MM, myeloma, plasma cell myeloma, or as Kahler's disease after Otto Kahler) is a cancer of plasma cells. These immune cells are formed in bone marrow, are numerous in lymphatics and produce antibodies. Myeloma is regarded as incurable, but remissions may be induced with steroids, chemotherapy, thalidomide and stem cell transplants. Myeloma is part of the broad group of diseases called hematological malignancies.

Multiple myeloma develops in post-germinal center B lymphocytes. A chromosomal translocation between the immunoglobulin heavy chain gene (on the fourteenth chromosome, locus 14q32) and an oncogene (often 11q13, 4p16.3, 6p21, 16q23 and 20q11) is frequently observed in patients with multiple myeloma. This mutation results in dysregulation of the oncogene which is thought to be an important initiating event in the pathogenesis of myeloma. The result is proliferation of a plasma cell clone and genomic instability that leads to further mutations and translocations. The chromosome 14 abnormality is observed in about 50% of all cases of myeloma. Deletion of (parts of) the thirteenth chromosome is also observed in about 50% of cases.

Production of cytokines (especially IL-6) by the plasma cells causes much of their localized damage, such as osteoporosis, and creates a microenvironment in which the malignant cells thrive. Angiogenesis (the attraction of new blood vessels) is increased.

In one case, the methods described herein treat a myeloma.

### Stomach cancer

Stomach or gastric cancer can develop in any part of the stomach and may spread throughout the stomach and to other organs; particularly the esophagus, lungs and the liver. Stomach cancer causes about 800.000 deaths worldwide per year.

Metastasis occurs in 80-90% of individuals with stomach cancer, with a six month survival rate of 65% in those diagnosed in early stages and less than 15% of those diagnosed in late stages.

Stomach cancer is often asymptomatic or causes only nonspecific symptoms in its early stages. By the time symptoms occur, the cancer has generally metastasized to other parts of the body, one of the main reasons for its poor prognosis.

In one case, the methods described herein treat a stomach cancer.

### Thyroid cancer

Thyroid neoplasm or thyroid cancer usually refers to any of four kinds of malignant tumors of the thyroid gland: papillary, follicular, medullary or anaplastic. Papillary and follicular tumors are the most common. They grow slowly and may recur, but are generally not fatal in patients under 45 years of age. Medullary tumors have a good prognosis if restricted to the thyroid gland and a poorer prognosis if metastasis occurs. Anaplastic tumors are fast-growing and respond poorly to therapy.

Thyroid cancer is usually found in a euthyroid patient, but symptoms of hyperthyroidism or hypothyroidism may be associated with a large or metastatic well-differentiated tumor. Nodules are of particular concern when they are found in those under the age of 20. The presentation of benign nodules at this age is less likely, and thus the potential for malignancy is far greater.

Thyroid cancers can be classified according to their pathological characteristics. The following variants can be distinguished (distribution over various subtypes may show regional variation): papillary thyroid cancer (up to 75%); follicular thyroid cancer (up to 15%); medullary thyroid cancer (up to 8%); and anaplastic thyroid cancer (less than 5%). The follicular and papillary types together can be classified as "differentiated thyroid cancer". These types have a more favorable prognosis than the medullary and undifferentiated types. Thyroid adenoma is a benign neoplasm of the thyroid.

In one case, the methods described herein treat a thyroid cancer.

### Bladder cancer

Bladder cancer refers to any of several types of malignant growths of the urinary bladder. It is a disease in which abnormal cells multiply without control in the bladder. The bladder is a hollow, muscular organ that stores urine; it is located in the pelvis. The most common type of bladder cancer begins in cells lining the inside of the bladder and is called transitional cell carcinoma (sometimes urothelial cell carcinoma).

90% of bladder cancers are transitional cell carcinoma. The other 10% are squamous cell carcinoma, adenocarcinoma, sarcoma, small cell carcinoma and secondary deposits from cancers elsewhere in the body.

The following stages are used to classify the location, size, and spread of the cancer, according to the TNM (tumor, lymph node, and metastasis) staging system: Stage 0: Cancer cells are found only on the inner lining of the bladder. Stage I: Cancer cells have proliferated to the layer beyond the inner lining of the urinary bladder but not to the muscles of the urinary bladder. Stage II: Cancer cells have proliferated to the muscles in the bladder wall but not to the fatty tissue that surrounds the urinary bladder. Stage III: Cancer cells have proliferated to the fatty tissue surrounding the urinary bladder and to the prostate gland, vagina, or uterus, but not to the lymph nodes or other organs. Stage IV: Cancer cells have proliferated to the lymph nodes, pelvic or abdominal wall, and/or other organs. Recurrent: Cancer has recurred in the urinary bladder or in another nearby organ after having been treated.

Bladder TCC is staged according to the 1997 TNM system: Ta Non-invasive papillary tumor; T1 Invasive but not as far as the muscular bladder layer; T2 Invasive into the muscular layer; T3 Invasive beyond the muscle into the fat outside the bladder; and T4 Invasive into surrounding structures like the prostate, uterus or pelvic wall.

In one case, the methods described herein treat a bladder cancer.

### Combinations

In certain cases, the combination of anticancer therapy with two BER pathway inhibitors (such as methoxyamine and a PARP inhibitor), and compositions thereof, is also used in further combination with other therapeutic agents that are selected for their therapeutic value for the condition to be treated. In some cases, it is appropriate to administer the anticancer therapy and the two BER pathway inhibitors described herein (such as methoxyamine and a PARP inhibitor) in combination with another therapeutic agent. By way of example only, if one of the side effects experienced by a patient upon receiving the combination of anticancer therapy with the two BER pathway inhibitors (such as methoxyamine and a PARP inhibitor) described herein is nausea, then it may be appropriate to administer an anti-nausea agent in combination with the anticancer agent and the two BER pathway inhibitors (such as methoxyamine and a PARP inhibitor) described herein. Or, by way of example only, the therapeutic effectiveness of the combination of anticancer therapy with two BER pathway inhibitors (such as methoxyamine and a PARP inhibitor) described herein may be enhanced by administration of an adjuvant (i.e., by itself the adjuvant may have minimal therapeutic benefit, but in combination with another therapeutic agent, the overall therapeutic benefit to the patient is enhanced). Or, by way of example only, the benefit experienced by a patient may be increased by administering the combination of anticancer therapy with two BER pathway inhibitors (such as methoxyamine and a PARP inhibitor) described herein with another therapeutic agent (which also includes a therapeutic regimen) that also has therapeutic benefit. In certain instances, the overall benefit experienced by the patient is simply additive of the therapeutic agents or the patient experiences a synergistic benefit.

In some cases, the combination of anticancer therapy with two BER pathway inhibitors (such as methoxyamine and a PARP inhibitor) described herein and, in cases where further combinational therapy is employed, other agents do not have to be administered in the same pharmaceutical composition, and are, because of different physical and chemical characteristics, have to be administered by different routes. The determination of the mode of administration and the advisability of administration, where possible, in the same pharmaceutical composition, is well within the knowledge of the clinician. In certain instances, the initial administration is made according to established protocols recognized in the field, and then, based upon the observed effects, the dosage, modes of administration and times of administration are modified by the clinician.

In some cases, the particular choice of route of administration and choice of treatment used depends upon the diagnosis of the attending physicians and their judgment of the condition of the patient and the appropriate treatment protocol. In certain cases, the combination of anticancer therapy with two BER inhibitors (such as methoxyamine and a PARP inhibitor) is administered concurrently (e.g., simultaneously, essentially simultaneously or within the same treatment protocol) or sequentially, depending upon the nature of the disease, disorder, or condition, the condition of the patient, and the actual choice of compounds used. In some instances, the determination of the order of administration of the combination of anticancer therapy and two BER pathway inhibitors (such as methoxyamine and a PARP inhibitor), and the number of repetitions of administration of each therapeutic agent and therapies during a treatment protocol, is well within the knowledge of the physician after evaluation of the disease being treated and the condition of the patient.

In some instances, therapeutically effective dosages vary when the drugs are used in treatment combinations. Methods for experimentally determining therapeutically effective dosages of drugs and other agents for use in combination treatment regimens are described in the literature. For example, the use of metronomic dosing, i.e., providing more frequent, lower doses in order to minimize toxic side effects, has been described extensively in the literature. In some cases, combination treatment further includes periodic treatments that start and stop at various times to assist with the clinical management of the patient.

In certain cases, the multiple therapeutic agents, which include the anticancer agent and the two BER pathway inhibitors (such as methoxyamine and a PARP inhibitor), are administered in any order or simultaneously. In some instances, if administered simultaneously, the multiple therapeutic agents are provided in a single, unified form, or in multiple forms (by way of example only, either as a single pill or as two separate pills). In certain instances, one of the therapeutic agents is given in multiple doses, or several of the therapeutic agents are given as multiple doses. In some instances, if not administered simultaneously, the timing between the multiple doses is about 1 day, about 2 days, about 3 days, about 4 days, about 5 days, about 6 days, about 7 days, about 10 days, about 2 weeks, about 3 weeks, about 4 weeks.

In some cases, for combinations of anticancer therapy with two BER pathway inhibitors (such as methoxyamine and a PARP inhibitor) described herein, dosages of the co-administered compounds will of course vary depending on the type of co-drug employed, on the specific drug employed, on the disease or condition being treated and so forth. In certain cases, when the combination of anticancer therapy with two BER pathway inhibitors (such as methoxyamine and a PARP inhibitor) described herein is co-administered with one or more biologically active agents, the compounds of the combination provided herein are administered either simultaneously with the biologically active agent(s), or sequentially. In some instances, if administered sequentially, the attending physician will decide on the appropriate sequence of administering combination of anticancer therapy with two BER pathway inhibitors (such as methoxyamine and a PARP inhibitor) described herein in combination with the biologically active agent(s).

In certain instances, the dosage regimen to treat, prevent, or ameliorate the cancer, is modified in accordance with a variety of factors. These factors include the disorder or condition from which the subject suffers, as well as the age, weight, sex, diet, and medical condition of the subject. Thus, in certain instances, the dosage regimen actually employed varies widely.

In some cases, the pharmaceutical agents that make up the combination of anticancer therapy with two BER pathway inhibitors (such as methoxyamine and a PARP inhibitor) described herein are present in a combined dosage form or in separate dosage forms intended for substantially simultaneous administration. In certain cases, the pharmaceutical agents that make up the combination of anticancer therapy with two BER pathway inhibitors (such as methoxyamine and a PARP inhibitor) described herein are administered sequentially, with either therapeutic compound being administered by a regimen calling for two-step administration. In some cases, the two-step administration regimen calls for sequential administration of the active agents or spaced-apart administration of the separate active agents. In certain cases, the time period between the multiple administration steps ranges from a few minutes to several hours, depending upon the properties of each pharmaceutical agent, such as potency, solubility, bioavailability, plasma half-life and kinetic profile of the pharmaceutical agent. In specific instances, the circadian variation of the target molecule concentration also determines the optimal dose interval.

In some cases, the combination of anticancer therapy with two BER pathway inhibitors (such as methoxyamine and a PARP inhibitor) as described herein is used in combination with procedures that provide additional or synergistic benefit to the patient. By way of example only, patients are expected to find therapeutic benefit when pharmaceutical composition or method of treatment comprising a combination of anticancer therapy with two BER pathway inhibitors (such as methoxyamine and a PARP inhibitor) described herein are combined with genetic testing to determine whether that individual is a carrier of a mutant gene that is known to be correlated with certain diseases or conditions.

In certain cases, the anticancer agent and/or the two BER pathway inhibitors (such as methoxyamine and a PARP inhibitor) are administered orally, parenterally, directly to the tumor, transdermally, buccally, or a combination thereof. In some cases, the anticancer agent is administered orally, parenterally, directly to the tumor, transdermally, or buccally. In some cases, the AP site binder is administered orally, parenterally, directly to the tumor, transdermally, or buccally. In certain cases, methoxyamine is administered orally, parenterally, directly to the tumor, transdermally, or buccally. In some cases, a PARP inhibitor is administered orally, parenterally, directly to the tumor, transdermally, or buccally. In certain cases, ABT-888 is administered orally, parenterally, directly to the tumor, transdermally, or buccally. In some cases, the individual agents are co-formulated in the same dosage form. In certain cases, the anticancer agent is a co-formulated with the AP site binder. In some cases, the anticancer agent is co-formulated with the PARP inhibitor. In certain cases, the AP site binder is co-formulated with the PARP inhibitor. In some cases, the anticancer agent is co-formulated with the two BER inhibitors.

In some cases, an agent, such as any combination of anticancer therapy with two BER pathway inhibitors (such as methoxyamine and a PARP inhibitor) as described herein, is administered in an amount effective for amelioration of symptoms of the disease or disorder (i.e., a therapeutically effective amount). In specific cases, a therapeutically effective amount is an amount that is capable of at least partially reversing a disease or disorder. In certain instances, the dose required to obtain an effective amount varies depending on the agent, formulation, disease or disorder, and individual to whom the agent is administered.

In some instances, determination of effective amounts also involves *in vitro* assays in which varying doses of agent are administered to cells in culture and the concentration of agent effective for ameliorating some or all symptoms is determined in order to calculate the concentration required *in vivo*. In certain instances, effective amounts also are based on *in vivo* animal studies.

In certain instances, the compositions, such as any combination of anticancer therapy with two BER pathway inhibitors (such as methoxyamine and a PARP inhibitor) as described herein, are administered to a patient already suffering from a disease or condition, in an amount sufficient to cure or at least partially arrest the symptoms of the disease or condition. Amounts effective for this use will depend on the severity and course of the disease or condition, previous therapy, the patient's health status, weight, and response to the drugs, and the judgment of the treating physician.

In some instances, the amount of a given agent of a combination of anticancer therapy with two BER pathway inhibitors (such as methoxyamine and a PARP inhibitor) will vary depending upon factors such as the particular compound, disease or condition and its severity, the identity (e.g., weight) of the subject or host in need of treatment, but nevertheless is determined in a manner recognized in the field according to the particular circumstances surrounding the case, including, e.g., the specific agent being administered, the route of administration, the condition being treated, and the subject or host being treated. In certain instances, doses employed for adult human treatment will typically be in the range of about 0.02 mg to about 5000 mg per day, in some cases, about 1 - about 1500 mg per day. In one case, about 10 - about 1000 mg per day. In another case, about 50 - about 750 mg per day. In yet another case, about 100 - 500 mg per day. In a further case, about 250 - 400 mg per day. In some instances, the desired dose is conveniently be presented in a single dose or as divided doses administered simultaneously (or over a short period of time) or at appropriate intervals, for example as two, three, four or more sub-doses per day.

In certain instances, toxicity and therapeutic efficacy of any combination of anticancer therapy with two BER pathway inhibitors (such as methoxyamine and a PARP inhibitor) is determined by standard pharmaceutical procedures in cell cultures or experimental animals, including, but not limited to, the determination of the LD₅₀ (the dose lethal to 50% of the population) and the ED₅₀ (the dose therapeutically effective in 50% of the population). In specific instances, the dose ratio between the toxic and therapeutic effects is the therapeutic index and it is expressed as the ratio between LD₅₀ and ED₅₀. Compounds exhibiting high therapeutic indices are preferred. In some instances, the data obtained from cell culture assays and animal studies are used in formulating a range of dosage for use in human. In certain instances, the dosage of such compounds lies preferably within a range of circulating concentrations that include the ED₅₀ while displaying minimal toxicity.

In certain cases, the dose of an alkylating anticancer agent, e.g., temozolomide, is about 1 mg/m² per day, is about 2 mg/m² per day, is about 5 mg/m² per day, is about 10 mg/m² per day, is about 15 mg/m² per day, is about 20 mg/m² per day, is about 25 mg/m² per day, is about 30 mg/m² per day, is about 35 mg/m² per day, is about 40 mg/m² per day, is about 45 mg/m² per day, is about 50 mg/m² per day, is about 55 mg/m² per day, is about 60 mg/m² per day, is about 65 mg/m² per day, is about 70 mg/m² per day, is about 75 mg/m² per day, is about 80 mg/m² per day, is about 85 mg/m² per day, is about 90 mg/m² per day, is about 100 mg/m² per day, is about 110 mg/m² per day, is about 120 mg/m² per day, is about 130 mg/m² per day, is about 140 mg/m² per day, is about 150 mg/m² per day, is about 160 mg/m² per day, is about 170 mg/m² per day, is about 180 mg/m² per day, is about 190 mg/m² per day, is about 200 mg/m² per day, is about 210 mg/m² per day, is about 220mg/m² per day, is about 230 mg/m² per day, is about 240 mg/m² per day, or is about 250 mg/m² per day. In some cases, the dose of an alkylating anticancer agent, e.g., temozolomide, is less than 1 mg/m² per day, is less than 2 mg/m² per day, is less than 5 mg/m² per day, is less than 10 mg/m² per day, is less than 15 mg/m² per day, is less than 20 mg/m² per day, is less than 25 mg/m² per day, is less than 30 mg/m² per day, is less than 35 mg/m² per day, is less than 40 mg/m² per day, is less than 45 mg/m² per day, is less than 50 mg/m² per day, is less than 55 mg/m² per day, is less than 60 mg/m² per day, is less than 65 mg/m² per day, is less than 70 mg/m² per day, is less than 75 mg/m² per day, is less than 80 mg/m² per day, is less than 85 mg/m² per day, is less than 90 mg/m² per day, is less than 100 mg/m² per day, is less than 110 mg/m² per day, is less than 120 mg/m² per day, is less than 130 mg/m² per day, is less than 140 mg/m² per day, is less than 150 mg/m² per day, is less than 160 mg/m² per day, is less than 170 mg/m² per day, is less than 180 mg/m² per day, is less than 190 mg/m² per day, is less than 200 mg/m² per day, is less than 210 mg/m² per day, is less than 220mg/m² per day, is less than 230 mg/m² per day, is less than 240 mg/m² per day, or is less than 250 mg/m² per day. In some cases, the dose of an alkylating anticancer agent, e.g., temozolomide, is more than 1 mg/m² per day, is more than 2 mg/m² per day, is more than 5 mg/m² per day, is more than 10 mg/m² per day, is more than 15 mg/m² per day, is more than 20 mg/m² per day, is more than 25 mg/m² per day, is more than 30 mg/m² per day, is more than 35 mg/m² per day, is more than 40 mg/m² per day, is more than 45 mg/m² per day, is more than 50 mg/m² per day, is more than 55 mg/m² per day, is more than 60 mg/m² per day, is more than 65 mg/m² per day, is more than 70 mg/m² per day, is more than 75 mg/m² per day, is more than 80 mg/m² per day, is more than 85 mg/m² per day, is more than 90 mg/m² per day, is more than 100 mg/m² per day, is more than 110 mg/m² per day, is more than 120 mg/m² per day, is more than 130 mg/m² per day, is more than 140 mg/m² per day, is more than 150 mg/m² per day, is more than 160 mg/m² per day, is more than 170 mg/m² per day, is more than 180 mg/m² per day, is more than 190 mg/m² per day, is more than 200 mg/m² per day, is more than 210 mg/m² per day, is more than 220mg/m² per day, is more than 230 mg/m² per day, is more than 240 mg/m² per day, or is more than 250 mg/m² per day. In certain cases, the dose of an alkylating anticancer agent, e.g., temozolomide, is more than 25 mg/m² per day and less than 100 mg/m² per day. In some cases, the dose of an alkylating anticancer agent, e.g., temozolomide, is more than 1 mg/m² per day and less than 50 mg/m² per day. In some cases, the dose of an alkylating anticancer agent, e.g., temozolomide, is more than 100 mg/m² per day and less than 250 mg/m² per day.

In some cases, the dose of an alkylating anticancer agent, e.g., pemetrexed, is about 10 mg/m² per day, is about 25 mg/m² per day, is about 50 mg/m² per day, is about 75 mg/m² per day, is about 100 mg/m² per day, is about 125 mg/m² per day, is about 150 mg/m² per day, is about 175 mg/m² per day, is about 200 mg/m² per day, is about 225 mg/m² per day, is about 250 mg/m² per day, is about 275 mg/m² per day, is about 300 mg/m² per day, is about 325 mg/m² per day, is about 350 mg/m² per day, is about 375 mg/m² per day, is about 400 mg/m² per day, is about 425 mg/m² per day, is about 450 mg/m² per day, is about 475 mg/m² per day, is about 500 mg/m² per day, is about 525 mg/m² per day, is about 550 mg/m² per day, is about 575 mg/m² per day, is about 600 mg/m² per day, is about 650 mg/m² per day, is about 700 mg/m² per day, is about 800 mg/m² per day, is about 900 mg/m² per day, or is about 1000 mg/m² per day. In certain cases, the dose of an alkylating anticancer agent, e.g., pemetrexed, is less than 10 mg/m² per day, is less than 25 mg/m² per day, is less than 50 mg/m² per day, is less than 75 mg/m² per day, is less than 100 mg/m² per day, is less than 125 mg/m² per day, is less than 150 mg/m² per day, is less than 175 mg/m² per day, is less than 200 mg/m² per day, is less than 225 mg/m² per day, is less than 250 mg/m² per day, is less than 275 mg/m² per day, is less than 300 mg/m² per day, is less than 325 mg/m² per day, is less than 350 mg/m² per day, is less than 375 mg/m² per day, is less than 400 mg/m² per day, is less than 425 mg/m² per day, is less than 450 mg/m² per day, is less than 475 mg/m² per day, is less than 500 mg/m² per day, is less than 525 mg/m² per day, is less than 550 mg/m² per day, is less than 575 mg/m² per day, is less than 600 mg/m² per day, is less than 650 mg/m² per day, is less than 700 mg/m² per day, is less than 800 mg/m² per day, is less than 900 mg/m² per day, or is less than 1000 mg/m² per day. In some cases, the dose of an alkylating anticancer agent, e.g., pemetrexed, is more than 10 mg/m² per day, is more than 25 mg/m² per day, is more than 50 mg/m² per day, is more than 75 mg/m² per day, is more than 100 mg/m² per day, is more than 125 mg/m² per day, is more than 150 mg/m² per day, is more than 175 mg/m² per day, is more than 200 mg/m² per day, is more than 225 mg/m² per day, is more than 250 mg/m² per day, is more than 275 mg/m² per day, is more than 300 mg/m² per day, is more than 325 mg/m² per day, is more than 350 mg/m² per day, is more than 375 mg/m² per day, is more than 400 mg/m² per day, is more than 425 mg/m² per day, is more than 450 mg/m² per day, is more than 475 mg/m² per day, is more than 500 mg/m² per day, is more than 525 mg/m² per day, is more than 550 mg/m² per day, is more than 575 mg/m² per day, is more than 600 mg/m² per day, is more than 650 mg/m² per day, is more than 700 mg/m² per day, is more than 800 mg/m² per day, is more than 900 mg/m² per day, or is more than 1000 mg/m² per day. In some cases, the dose of an alkylating anticancer agent, e.g., pemetrexed, is more than 200 mg/m² per day and less than 500 mg/m² per day. In certain cases, the dose of an alkylating anticancer agent, e.g., pemetrexed, is more than 10 mg/m² per day and less than 200 mg/m² per day. In some cases, the dose of an alkylating anticancer agent, e.g., pemetrexed, is more than 150 mg/m² per day and less than 800 mg/m² per day.

In certain cases, the dose of an AP site binder, e.g., methoxyamine, is about 1 mg/m² per day, is about 2 mg/m² per day, is about 5 mg/m² per day, is about 10 mg/m² per day, is about 20 mg/m² per day, is about 25 mg/m² per day, is about 30 mg/m² per day, is about 35 mg/m² per day, is about 40 mg/m² per day, is about 45 mg/m² per day, is about 50 mg/m² per day, is about 55 mg/m² per day, is about 60 mg/m² per day, is about 65 mg/m² per day, is about 70 mg/m² per day, is about 75 mg/m² per day, is about 80 mg/m² per day, is about 85 mg/m² per day, is about 90 mg/m² per day, is about 100 mg/m² per day, is about 110 mg/m² per day, is about 120 mg/m² per day, is about 130 mg/m² per day, is about 140 mg/m² per day, or is about 150 mg/m² per day. In some cases, the dose of an AP site binder, e.g., methoxyamine, is less than 1 mg/m² per day, is less than 2 mg/m² per day, is less than 5 mg/m² per day, is less than 10 mg/m² per day, is less than 20 mg/m² per day, is less than 25 mg/m² per day, is less than 30 mg/m² per day, is less than 35 mg/m² per day, is less than 40 mg/m² per day, is less than 45 mg/m² per day, is less than 50 mg/m² per day, is less than 55 mg/m² per day, is less than 60 mg/m² per day, is less than 65 mg/m² per day, is less than 70 mg/m² per day, is less than 75 mg/m² per day, is less than 80 mg/m² per day, is less than 85 mg/m² per day, is less than 90 mg/m² per day, is less than 100 mg/m² per day, is less than 110 mg/m² per day, is less than 120 mg/m² per day, is less than 130 mg/m² per day, is less than 140 mg/m² per day, or is less than 150 mg/m² per day. In certain cases, the dose of an AP site binder, e.g., methoxyamine, is more than 1 mg/m² per day, is more than 2 mg/m² per day, is more than 5 mg/m² per day, is more than 10 mg/m² per day, is more than 20 mg/m² per day, is more than 25 mg/m² per day, is more than 30 mg/m² per day, is more than 35 mg/m² per day, is more than 40 mg/m² per day, is more than 45 mg/m² per day, is more than 50 mg/m² per day, is more than 55 mg/m² per day, is more than 60 mg/m² per day, is more than 65 mg/m² per day, is more than 70 mg/m² per day, is more than 75 mg/m² per day, is more than 80 mg/m² per day, is more than 85 mg/m² per day, is more than 90 mg/m² per day, is more than 100 mg/m² per day, is more than 110 mg/m² per day, is more than 120 mg/m² per day, is more than 130 mg/m² per day, is more than 140 mg/m² per day, or is more than 150 mg/m² per day. In some cases, the dose of an AP site binder, e.g., methoxyamine, is more than 5 mg/m² per day and less than 100 mg/m² per day. In certain cases, the dose of an AP site binder, e.g., methoxyamine, is more than 1 mg/m² per day and less than 20 mg/m² per day. In some cases, the dose of an AP site binder, e.g., methoxyamine, is more than 50 mg/m² per day and less than 150 mg/m² per day.

In certain cases, the dose of a PARP inhibitor, e.g., ABT-888, is about 0.5 mg/kg per day, is about 1 mg/kg per day, is about 2 mg/kg per day, is about 5 mg/kg per day, is about 10 mg/kg per day, is about 15 mg/kg per day, is about 20 mg/kg per day, is about 25 mg/kg per day, is about 30 mg/kg per day, is about 35 mg/kg per day, is about 40 mg/kg per day, is about 45 mg/kg per day, is about 50 mg/kg per day, is about 55 mg/kg per day, is about 60 mg/kg per day, is about 65 mg/kg per day, is about 70 mg/kg per day, is about 75 mg/kg per day, is about 80 mg/kg per day, is about 85 mg/kg per day, is about 90 mg/kg per day, is about 100 mg/kg per day, is about 110 mg/kg per day, is about 120 mg/kg per day, is about 130 mg/kg per day, is about 140 mg/kg per day, or is about 150 mg/kg per day. In some cases, the dose of a PARP inhibitor, e.g., ABT-888, is less than 0.5 mg/kg per day, is less than 1 mg/kg per day, is less than 2 mg/kg per day, is less than 5 mg/kg per day, is less than 10 mg/kg per day, is less than 15 mg/kg per day, is less than 20 mg/kg per day, is less than 25 mg/kg per day, is less than 30 mg/kg per day, is less than 35 mg/kg per day, is less than 40 mg/kg per day, is less than 45 mg/kg per day, is less than 50 mg/kg per day, is less than 55 mg/kg per day, is less than 60 mg/kg per day, is less than 65 mg/kg per day, is less than 70 mg/kg per day, is less than 75 mg/kg per day, is less than 80 mg/kg per day, is less than 85 mg/kg per day, is less than 90 mg/kg per day, is less than 100 mg/kg per day, is less than 110 mg/kg per day, is less than 120 mg/kg per day, is less than 130 mg/kg per day, is less than 140 mg/kg per day, or is less than 150 mg/kg per day. In certain cases, the dose of a PARP inhibitor, e.g., ABT-888, is more than 0.5 mg/kg per day, is more than 1 mg/kg per day, is more than 2 mg/kg per day, is more than 5 mg/kg per day, is more than 10 mg/kg per day, is more than 15 mg/kg per day, is more than 20 mg/kg per day, is more than 25 mg/kg per day, is more than 30 mg/kg per day, is more than 35 mg/kg per day, is more than 40 mg/kg per day, is more than 45 mg/kg per day, is more than 50 mg/kg per day, is more than 55 mg/kg per day, is more than 60 mg/kg per day, is more than 65 mg/kg per day, is more than 70 mg/kg per day, is more than 75 mg/kg per day, is more than 80 mg/kg per day, is more than 85 mg/kg per day, is more than 90 mg/kg per day, is more than 100 mg/kg per day, is more than 110 mg/kg per day, is more than 120 mg/kg per day, is more than 130 mg/kg per day, is more than 140 mg/kg per day, or is more than 150 mg/kg per day. In some cases, the dose of a PARP inhibitor, e.g., ABT-888, is more than 1 mg/kg per day and less than 50 mg/kg per day. In certain cases, the dose of a PARP inhibitor, e.g., ABT-888, is more than 0.5 mg/kg per day and less than 20 mg/kg per day. In some cases, the dose of a PARP inhibitor, e.g., ABT-888, is more than 50 mg/kg per day and less than 150 mg/kg per day,

In certain cases, the dose of a radiation therapy is about 1 Gy, about 2 Gy, about 5 Gy, about 10 Gy, about 15 Gy, about 20 Gy, about 25 Gy, about 30 Gy, about 35 Gy, about 40 Gy, about 45 Gy, about 50 Gy, about 55 Gy, about 60 Gy, about 65 Gy, about 70 Gy, about 75 Gy, about 80 Gy, about 90 Gy, or about 100 Gy. In some cases, the dose of a radiation therapy is less than 1 Gy, less than 2 Gy, less than 5 Gy, less than 10 Gy, less than 15 Gy, less than 20 Gy, less than 25 Gy, less than 30 Gy, less than 35 Gy, less than 40 Gy, less than 45 Gy, less than 50 Gy, less than 55 Gy, less than 60 Gy, less than 65 Gy, less than 70 Gy, less than 75 Gy, less than 80 Gy, less than 90 Gy, or less than 100 Gy. In certain cases, the dose of a radiation therapy is more than 1 Gy, more than 2 Gy, more than 5 Gy, more than 10 Gy, more than 15 Gy, more than 20 Gy, more than 25 Gy, more than 30 Gy, more than 35 Gy, more than 40 Gy, more than 45 Gy, more than 50 Gy, more than 55 Gy, more than 60 Gy, more than 65 Gy, more than 70 Gy, more than 75 Gy, more than 80 Gy, more than 90 Gy, or more than 100 Gy. In some cases, the dose of a radiation therapy is more than 20 Gy and less than 60 Gy. In certain cases, the dose of a radiation therapy is more than 40 Gy and less than 80 Gy. In some cases, the dose of a radiation therapy is more than 1 Gy and less than 50 Gy.

### General Definitions

The term "subject", "patient" or "individual" are used interchangeably herein and refer to mammals and non-mammals, e.g., suffering from a disorder described herein. Examples of mammals include, but are not limited to, any member of the Mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. Examples of non-mammals include, but are not limited to, birds, fish and the like. In one case of the methods and compositions provided herein, the mammal is a human.

The terms "treat," "treating" or "treatment," and other grammatical equivalents as used herein, include alleviating, inhibiting or reducing symptoms, reducing or inhibiting severity of, reducing incidence of, prophylactic treatment of, reducing or inhibiting recurrence of, delaying onset of, delaying recurrence of, abating or ameliorating a disease or condition symptoms, ameliorating the underlying metabolic causes of symptoms, inhibiting the disease or condition, e.g., arresting the development of the disease or condition, relieving the disease or condition, causing regression of the disease or condition, relieving a condition caused by the disease or condition, or stopping the symptoms of the disease or condition. The terms further include achieving a therapeutic benefit. By therapeutic benefit is meant eradication or amelioration of the underlying disorder being treated, and/or the eradication or amelioration of one or more of the physiological symptoms associated with the underlying disorder such that an improvement is observed in the patient.

The terms "prevent," "preventing" or "prevention," and other grammatical equivalents as used herein, include preventing additional symptoms, preventing the underlying metabolic causes of symptoms, inhibiting the disease or condition, e.g., arresting the development of the disease or condition and are intended to include prophylaxis. The terms further include achieving a prophylactic benefit. For prophylactic benefit, the compositions are optionally administered to a patient at risk of developing a particular disease, to a patient reporting one or more of the physiological symptoms of a disease, or to a patient at risk of reoccurrence of the disease.

The terms "effective amount" or "therapeutically effective amount" as used herein, refer to a sufficient amount of at least one agent being administered which achieve a desired result, e.g., to relieve to some extent one or more symptoms of a disease or condition being treated. In certain instances, the result is a reduction and/or alleviation of the signs, symptoms, or causes of a disease, or any other desired alteration of a biological system. In certain instances, an "effective amount" for therapeutic uses is the amount of the composition comprising an agent as set forth herein required to provide a clinically significant decrease in a disease. An appropriate "effective" amount in any individual case is determined using any suitable technique, such as a dose escalation study.

The terms "administer," "administering", "administration," and the like, as used herein, refer to the methods that are used to enable delivery of agents or compositions to the desired site of biological action. These methods include, but are not limited to oral routes, intraduodenal routes, parenteral injection (including intravenous, subcutaneous, intraperitoneal, intramuscular, intravascular or infusion), topical and rectal administration. Administration techniques that in some instances are employed with the agents and methods described herein include, e.g., as discussed in Goodman and Gilman, The Pharmacological Basis of Therapeutics (current edition), Pergamon; and Remington's, Pharmaceutical Sciences (current edition), Mack Publishing Co., Easton, Pa. In certain cases, the agents and compositions described herein are administered orally. In some cases, the compositions described herein are administered parenterally.

The term "pharmaceutically acceptable" as used herein, refers to a material that does not abrogate the biological activity or properties of the agents described herein, and is relatively nontoxic (i.e., the toxicity of the material significantly outweighs the benefit of the material). In some instances, a pharmaceutically acceptable material is administered to an individual without causing significant undesirable biological effects or significantly interacting in a deleterious manner with any of the components of the composition in which it is contained.

### Further Forms of Compounds

The methods and compositions described herein include the use of amorphous forms as well as crystalline forms (also known as polymorphs). In some instances, the compounds described herein are in the form of pharmaceutically acceptable salts. As well, active metabolites of these compounds having the same type of activity are included in the scope of the present disclosure. In other instances, the compounds described herein exist in unsolvated as well as solvated forms with pharmaceutically acceptable solvents such as water, ethanol, and the like. The solvated forms of the compounds presented herein are also considered to be disclosed herein.

Prodrug forms of the herein described compounds, wherein the prodrug is metabolized *in vivo* to produce any of the anticancer agent or the two BER pathway inhibitors (such as methoxyamine and a PARP inhibitor) as described herein, are included within the scope of the claims. In some cases, some of the herein-described compounds are a prodrug for another derivative or active compound.

Prodrugs are often useful because, in some instances, they are easier to administer than the parent drug. In certain instances, they are bioavailable by oral administration whereas the parent is not. In some cases, the prodrug also has improved solubility in pharmaceutical compositions over the parent drug. In some cases, prodrugs are designed as reversible drug derivatives, for use as modifiers to enhance drug transport to site-specific tissues. In some cases, the design of a prodrug increases the effective water solubility. See, e.g., Fedorak et al., Am. J. Physiol., 269:G210-218 (1995); McLoed et al., Gastroenterol, 106:405-413 (1994); Hochhaus et al., Biomed. Chrom., 6:283-286 (1992); J. Larsen and H. Bundgaard, Int. J. Pharmaceutics, 37, 87 (1987); J. Larsen et al., Int. J. Pharmaceutics, 47, 103 (1988); Sinkula et al., J. Pharm. Sci., 64:181-210 (1975); T. Higuchi and V. Stella, Pro-drugs as Novel Delivery Systems, Vol. 14 of the A.C.S. Symposium Series; and Edward B. Roche, Bioreversible Carriers in Drug Design, American Pharmaceutical Association and Pergamon Press, 1987, all incorporated herein for such disclosure).

In some instances, the compounds described herein are labeled isotopically (e.g. with a radioisotope) or by other means, including, but not limited to, the use of chromophores or fluorescent moieties, bioluminescent labels, photoactivatable or chemiluminescent labels.

Compounds described herein include isotopically-labeled compounds, which are identical to those recited herein, but for the fact that one or more atoms are replaced by an atom having an atomic mass or mass number different from the atomic mass or mass number usually found in nature. Examples of isotopes that can be incorporated into the present compounds include isotopes of hydrogen, carbon, nitrogen, oxygen, fluorine and chlorine, such as, for example, ²H, ³H, ¹³C, ¹⁴C, ¹⁵N, ¹⁸O, ¹⁷O, ³⁵S, ¹⁸F, ³⁶Cl respectively. Certain isotopically-labeled compounds described herein, for example those into which radioactive isotopes such as ³H and ¹⁴C are incorporated, are useful in drug and/or substrate tissue distribution assays. In some instances, substitution with isotopes such as deuterium, i.e., ²H, affords certain therapeutic advantages resulting from greater metabolic stability, such as, for example, increased *in vivo* half-life or reduced dosage requirements.

In additional or further cases, the compounds described herein are metabolized upon administration to an organism in need to produce a metabolite that is then used to produce a desired effect, including a desired therapeutic effect.

In some instances, compounds described herein are formed as, and/or used as, pharmaceutically acceptable salts. The type of pharmaceutical acceptable salts, include, but are not limited to: (1) acid addition salts, formed by reacting the free base form of the compound with a pharmaceutically acceptable: inorganic acid, such as, for example, hydrochloric acid, hydrobromic acid, sulfuric acid, phosphoric acid, metaphosphoric acid, and the like; or with an organic acid, such as, for example, acetic acid, propionic acid, hexanoic acid, cyclopentanepropionic acid, glycolic acid, pyruvic acid, lactic acid, malonic acid, succinic acid, malic acid, maleic acid, fumaric acid, trifluoroacetic acid, tartaric acid, citric acid, benzoic acid, 3-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, toluenesulfonic acid, 2-naphthalenesulfonic acid, 4-methylbicyclo-[2.2.2]oct-2-ene-1-carboxylic acid, glucoheptonic acid, 4,4'-methylenebis-(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, butyric acid, phenylacetic acid, phenylbutyric acid, valproic acid, and the like; (2) salts formed when an acidic proton present in the parent compound is replaced by a metal ion, e.g., an alkali metal ion (e.g. lithium, sodium, potassium), an alkaline earth ion (e.g. magnesium, or calcium), or an aluminum ion. In some cases, compounds described herein coordinate with an organic base, such as, but not limited to, ethanolamine, diethanolamine, triethanolamine, tromethamine, N-methylglucamine, dicyclohexylamine, tris(hydroxymethyl)methylamine. In other cases, compounds described herein form salts with amino acids such as, but not limited to, arginine, lysine, and the like. Acceptable inorganic bases used to form salts with compounds that include an acidic proton, include, but are not limited to, aluminum hydroxide, calcium hydroxide, potassium hydroxide, sodium carbonate, sodium hydroxide, and the like.

It should be understood that a reference to a pharmaceutically acceptable salt includes the solvent addition forms or crystal forms thereof, particularly solvates or polymorphs. Solvates contain either stoichiometric or non-stoichiometric amounts of a solvent, and are formed during the process of crystallization with pharmaceutically acceptable solvents such as water, ethanol, and the like. Hydrates are formed when the solvent is water, or alcoholates are formed when the solvent is alcohol. In some instances, solvates of compounds described herein are conveniently prepared or formed during the processes described herein. In other instances, the compounds provided herein exist in unsolvated as well as solvated forms. In general, the solvated forms are considered equivalent to the unsolvated forms for the purposes of the compounds and methods provided herein.

In some cases, compounds described herein, such as any combination of an anticancer agent with two BER pathway inhibitors (such as methoxyamine and a PARP inhibitor) as described herein, are in various forms, including but not limited to, amorphous forms, milled forms and nano-particulate forms. In addition, compounds described herein include crystalline forms, also known as polymorphs. Polymorphs include the different crystal packing arrangements of the same elemental composition of a compound. Polymorphs usually have different X-ray diffraction patterns, melting points, density, hardness, crystal shape, optical properties, stability, and solubility. In certain instances, various factors such as the recrystallization solvent, rate of crystallization, and storage temperature cause a single crystal form to dominate.

In some instances, the screening and characterization of the pharmaceutically acceptable salts, polymorphs and/or solvates is accomplished using a variety of techniques including, but not limited to, thermal analysis, x-ray diffraction, spectroscopy, vapor sorption, and microscopy. Thermal analysis methods address thermo chemical degradation or thermo physical processes including, but not limited to, polymorphic transitions, and such methods are used to analyze the relationships between polymorphic forms, determine weight loss, to find the glass transition temperature, or for excipient compatibility studies. Such methods include, but are not limited to, Differential scanning calorimetry (DSC), Modulated Differential Scanning Calorimetry (MDCS), Thermogravimetric analysis (TGA), and Thermogravi-metric and Infrared analysis (TG/IR). X-ray diffraction methods include, but are not limited to, single crystal and powder diffractometers and synchrotron sources. The various spectroscopic techniques used include, but are not limited to, Raman, FTIR, UV-VIS, and NMR (liquid and solid state). The various microscopy techniques include, but are not limited to, polarized light microscopy, Scanning Electron Microscopy (SEM) with Energy Dispersive X-Ray Analysis (EDX), Environmental Scanning Electron Microscopy with EDX (in gas or water vapor atmosphere), IR microscopy, and Raman microscopy.

### Pharmaceutical Compositions and Methods of Administration

In some cases, pharmaceutical compositions are formulated in a conventional manner using one or more physiologically acceptable carriers including excipients and auxiliaries which facilitate processing of the active compounds into preparations which are used pharmaceutically. Proper formulation is dependent upon the route of administration chosen. Additional details about suitable excipients for pharmaceutical compositions described herein are found, for example, in Remington, The Science and Practice of Pharmacy, Nineteenth Ed (Easton, Pa.: Mack Publishing Company, 1995); Hoover, John E., Remington's Pharmaceutical Sciences, Mack Publishing Co., Easton, Pennsylvania 1975; Liberman, H.A. and Lachman, L., Eds., Pharmaceutical Dosage Forms, Marcel Decker, New York, N.Y., 1980; and Pharmaceutical Dosage Forms and Drug Delivery Systems, Seventh Ed. (Lippincott Williams & Wilkins 1999), herein incorporated by reference for such disclosure.

A pharmaceutical composition, as used herein, refers to a mixture of an anticancer agent, a BER pathway inhibitor (such as methoxyamine or a PARP inhibitor), a combination of an anticancer agent with one or more BER pathway inhibitors, or a combination of two BER pathway inhibitors (such as methoxyamine and a PARP inhibitor), with other chemical components, such as carriers, stabilizers, diluents, dispersing agents, suspending agents, thickening agents, and/or excipients. The pharmaceutical composition facilitates administration of the compound to an organism. In practicing the methods of treatment or use provided herein, therapeutically effective amounts of compounds described herein are administered in a pharmaceutical composition to a mammal having a disease, disorder, or condition to be treated. In some cases, the mammal is a human. In certain instances, a therapeutically effective amount varies widely depending on the severity of the disease, the age and relative health of the subject, the potency of the compound used and other factors. In some instances, any combination of anticancer therapy with two BER pathway inhibitors (such as methoxyamine and a PARP inhibitor) as described herein is used in combination with one or more other therapeutic agents as components of mixtures (as in combination therapy).

In certain instances, pharmaceutical formulations described herein are administered to a subject by multiple administration routes, including but not limited to, oral, parenteral (e.g., intravenous, subcutaneous, intramuscular), intranasal, buccal, topical, rectal, or transdermal administration routes. In some cases, the pharmaceutical compositions described herein, which include any combination of anticancer therapy with two BER pathway inhibitors (such as methoxyamine and a PARP inhibitor) as described, is formulated into any suitable dosage form, including but not limited to, aqueous oral dispersions, liquids, gels, syrups, elixirs, slurries, suspensions, aerosols, controlled release formulations, fast melt formulations, effervescent formulations, lyophilized formulations, tablets, powders, pills, dragees, capsules, delayed release formulations, extended release formulations, pulsatile release formulations, multiparticulate formulations, and mixed immediate release and controlled release formulations.

In certain instances, the compounds and/or compositions are administered in a local rather than systemic manner, for example, via injection of the compound directly into an organ or tissue, often in a depot preparation or sustained release formulation. In some instances, such long acting formulations are administered by implantation (for example subcutaneously or intramuscularly) or by intramuscular injection. In other instances, the drug is administered in a targeted drug delivery system, for example, in a liposome coated with organ-specific antibody. The liposomes will be targeted to and taken up selectively by the organ. In further instances, the drug is provided in the form of a rapid release formulation, in the form of an extended release formulation, or in the form of an intermediate release formulation.

In some cases, pharmaceutical compositions including any combination of anticancer therapy with two BER pathway inhibitors (such as methoxyamine and a PARP inhibitor) as described herein are manufactured in a conventional manner, such as, by way of example only, by means of conventional mixing, dissolving, granulating, dragee-making, levigating, emulsifying, encapsulating, entrapping or compression processes.

The pharmaceutical compositions will include at least any combination of anticancer therapy with two BER pathway inhibitors (such as methoxyamine and a PARP inhibitor) as described herein, as active ingredients in free-acid or free-base form, or in a pharmaceutically acceptable salt form. In addition, the methods and pharmaceutical compositions described herein include the use of crystalline forms (also known as polymorphs), as well as active metabolites of these compounds having the same type of activity. In some situations, compounds exist as tautomers. All tautomers are included within the scope of the compounds presented herein. In other instances, the compounds described herein exist in unsolvated as well as solvated forms with pharmaceutically acceptable solvents such as water, ethanol, and the like. The solvated forms of the compounds presented herein are also considered to be disclosed herein.

In certain cases, compositions provided herein also include one or more preservatives to inhibit microbial activity. Suitable preservatives include quaternary ammonium compounds such as benzalkonium chloride, cetyltrimethylammonium bromide and cetylpyridinium chloride.

In some cases, pharmaceutical preparations for oral use are obtained by mixing one or more solid excipient with any combination of anticancer therapy and two BER pathway inhibitors (such as methoxyamine and a PARP inhibitor) as described herein, optionally grinding the resulting mixture, and processing the mixture of granules, after adding suitable auxiliaries, if desired, to obtain tablets, pills, or capsules. Suitable excipients include, for example, fillers such as sugars, including lactose, sucrose, mannitol, or sorbitol; cellulose preparations such as, for example, maize starch, wheat starch, rice starch, potato starch, gelatin, gum tragacanth, methylcellulose, microcrystalline cellulose, hydroxypropylmethylcellulose, sodium carboxymethylcellulose; or others such as: polyvinylpyrrolidone (PVP or povidone) or calcium phosphate. In certain instances, disintegrating agents are be added, such as the cross-linked croscarmellose sodium, polyvinylpyrrolidone, agar, or alginic acid or a salt thereof such as sodium alginate.

Dragee cores are provided with suitable coatings. In certain instances, concentrated sugar solutions are used for this purpose, which optionally contain gum arabic, talc, polyvinylpyrrolidone, carbopol gel, polyethylene glycol, and/or titanium dioxide, lacquer solutions, and suitable organic solvents or solvent mixtures. In some instances, dyestuffs or pigments are added to the tablets or dragee coatings for identification or to characterize different combinations of active compound doses.

In certain cases, pharmaceutical preparations that are used orally include push-fit capsules made of gelatin, as well as soft, sealed capsules made of gelatin and a plasticizer, such as glycerol or sorbitol. In some instances, the push-fit capsules contain the active ingredients in admixture with filler such as lactose, binders such as starches, and/or lubricants such as talc or magnesium stearate and, optionally, stabilizers. In certain instances, in soft capsules, the active compounds is dissolved or suspended in suitable liquids, such as fatty oils, liquid paraffin, or liquid polyethylene glycols. In specific instances, stabilizers are added.

In some cases, the solid dosage forms disclosed herein is in the form of a tablet, (including a suspension tablet, a fast-melt tablet, a bite-disintegration tablet, a rapid-disintegration tablet, an effervescent tablet, or a caplet), a pill, a powder (including a sterile packaged powder, a dispensable powder, or an effervescent powder), a capsule (including both soft or hard capsules, e.g., capsules made from animal-derived gelatin or plant-derived HPMC, or "sprinkle capsules"), solid dispersion, solid solution, bioerodible dosage form, controlled release formulations, pulsatile release dosage forms, multiparticulate dosage forms, pellets, granules, or an aerosol. In other cases, the pharmaceutical formulation is in the form of a powder. In still other cases, the pharmaceutical formulation is in the form of a tablet, including but not limited to, a fast-melt tablet. In some instances, pharmaceutical formulations of the compounds described herein are administered as a single capsule or in multiple capsule dosage form. In some cases, the pharmaceutical formulation is administered in two, or three, or four, capsules or tablets.

In some cases, solid dosage forms, e.g., tablets, effervescent tablets, and capsules, are prepared by mixing particles of any combination of anticancer therapy and two BER pathway inhibitors (such as methoxyamine and a PARP inhibitor) as described herein, with one or more pharmaceutical excipients to form a bulk blend composition. When referring to these bulk blend compositions as homogeneous, it is meant that the particles of any combination of anticancer therapy and two BER pathway inhibitors (such as methoxyamine and a PARP inhibitor) as described herein are dispersed evenly throughout the composition so that the composition is subdivided into equally effective unit dosage forms, such as tablets, pills, and capsules. In some instances, the individual unit dosages also include film coatings, which disintegrate upon oral ingestion or upon contact with diluent. In certain instances, these formulations are manufactured by conventional pharmacological techniques.

In some cases, the pharmaceutical solid dosage forms described herein includes any combination of anticancer therapy and two BER pathway inhibitors (such as methoxyamine and a PARP inhibitor) as described herein, and one or more pharmaceutically acceptable additives such as a compatible carrier, binder, filling agent, suspending agent, flavoring agent, sweetening agent, disintegrating agent, dispersing agent, surfactant, lubricant, colorant, diluent, solubilizer, moistening agent, plasticizer, stabilizer, penetration enhancer, wetting agent, anti-foaming agent, antioxidant, preservative, or one or more combination thereof. In still other aspects, using standard coating procedures, such as those described in Remington's Pharmaceutical Sciences, 20th Edition (2000), a film coating is provided around the formulation of the compound described herein. In one case, some or all of the particles of the compound described herein are coated. In another case, some or all of the particles of the compound described herein are microencapsulated. In still another case, the particles of the compound described herein are not microencapsulated and are uncoated.

Suitable carriers for use in the solid dosage forms described herein include, but are not limited to, acacia, gelatin, colloidal silicon dioxide, calcium glycerophosphate, calcium lactate, maltodextrin, glycerine, magnesium silicate, sodium caseinate, soy lecithin, sodium chloride, tricalcium phosphate, dipotassium phosphate, sodium stearoyl lactylate, carrageenan, monoglyceride, diglyceride, pregelatinized starch, hydroxypropylmethylcellulose, hydroxypropylmethylcellulose acetate stearate, sucrose, microcrystalline cellulose, lactose, mannitol and the like.

Suitable filling agents for use in the solid dosage forms described herein include, but are not limited to, lactose, calcium carbonate, calcium phosphate, dibasic calcium phosphate, calcium sulfate, microcrystalline cellulose, cellulose powder, dextrose, dextrates, dextran, starches, pregelatinized starch, hydroxypropylmethycellulose (HPMC), hydroxypropylmethycellulose phthalate, hydroxypropylmethylcellulose acetate stearate (HPMCAS), sucrose, xylitol, lactitol, mannitol, sorbitol, sodium chloride, polyethylene glycol, and the like.

In order to release any combination of anticancer therapy and two BER pathway inhibitors (such as methoxyamine and a PARP inhibitor) as described herein from a solid dosage form matrix as efficiently as possible, disintegrants are often used in the formulation, especially when the dosage forms are compressed with binder. Disintegrants help rupturing the dosage form matrix by swelling or capillary action when moisture is absorbed into the dosage form. Suitable disintegrants for use in the solid dosage forms described herein include, but are not limited to, natural starch such as corn starch or potato starch, a pregelatinized starch such as National 1551 or Amijel^{®}, or sodium starch glycolate such as Promogel^{®} or Explotab^{®}, a cellulose such as a wood product, methylcrystalline cellulose, e.g., Avicel^{®}, Avicel^{®} PH101, Avicel^{®} PH102, Avicel^{®} PH105, Elcema^{®} P100, Emcocel^{®}, Vivacel^{®}, Ming Tia^{®}, and Solka-Floc^{®}, methylcellulose, croscarmellose, or a cross-linked cellulose, such as cross-linked sodium carboxymethylcellulose (Ac-Di-Sol^{®}), cross-linked carboxymethylcellulose, or cross-linked croscarmellose, a cross-linked starch such as sodium starch glycolate, a cross-linked polymer such as crospovidone, a cross-linked polyvinylpyrrolidone, alginate such as alginic acid or a salt of alginic acid such as sodium alginate, a clay such as Veegum^{®} HV (magnesium aluminum silicate), a gum such as agar, guar, locust bean, Karaya, pectin, or tragacanth, sodium starch glycolate, bentonite, a natural sponge, a surfactant, a resin such as a cation-exchange resin, citrus pulp, sodium lauryl sulfate, sodium lauryl sulfate in combination starch, and the like.

In some instances, binders impart cohesiveness to solid oral dosage form formulations: for powder filled capsule formulation, they aid in plug formation that is filled into soft or hard shell capsules and for tablet formulation, they ensure the tablet remaining intact after compression and help assure blend uniformity prior to a compression or fill step. Materials suitable for use as binders in the solid dosage forms described herein include, but are not limited to, carboxymethylcellulose, methylcellulose (e.g., Methocel^{®}), hydroxypropylmethylcellulose (e.g. Hypromellose USP Pharmacoat-603, hydroxypropylmethylcellulose acetate stearate (Aqoate HS-LF and HS), hydroxyethylcellulose, hydroxypropylcellulose (e.g., Klucel^{®}), ethylcellulose (e.g., Ethocel^{®}), and microcrystalline cellulose (e.g., Avicel^{®}), microcrystalline dextrose, amylose, magnesium aluminum silicate, polysaccharide acids, bentonites, gelatin, polyvinylpyrrolidone/vinyl acetate copolymer, crospovidone, povidone, starch, pregelatinized starch, tragacanth, dextrin, a sugar, such as sucrose (e.g., Dipac^{®}), glucose, dextrose, molasses, mannitol, sorbitol, xylitol (e.g., Xylitab^{®}), lactose, a natural or synthetic gum such as acacia, tragacanth, ghatti gum, mucilage of isapol husks, starch, polyvinylpyrrolidone (e.g., Povidone^{®} CL, Kollidon^{®} CL, Polyplasdone^{®} XL-10, and Povidone^{®} K-12), larch arabogalactan, Veegum^{®}, polyethylene glycol, waxes, sodium alginate, and the like.

In general, binder levels of 20-70% are used in powder-filled gelatin capsule formulations. Binder usage level in tablet formulations varies whether direct compression, wet granulation, roller compaction, or usage of other excipients such as fillers which itself act as moderate binder. In some cases, formulators determine the binder level for the formulations, but binder usage level of up to 70% in tablet formulations is common.

Suitable lubricants or glidants for use in the solid dosage forms described herein include, but are not limited to, stearic acid, calcium hydroxide, talc, corn starch, sodium stearyl fumerate, alkali-metal and alkaline earth metal salts, such as aluminum, calcium, magnesium, zinc, stearic acid, sodium stearates, magnesium stearate, zinc stearate, waxes, Stearowet^{®}, boric acid, sodium benzoate, sodium acetate, sodium chloride, leucine, a polyethylene glycol or a methoxypolyethylene glycol such as Carbowax™, PEG 4000, PEG 5000, PEG 6000, propylene glycol, sodium oleate, glyceryl behenate, glyceryl palmitostearate, glyceryl benzoate, magnesium or sodium lauryl sulfate, and the like.

Suitable diluents for use in the solid dosage forms described herein include, but are not limited to, sugars (including lactose, sucrose, and dextrose), polysaccharides (including dextrates and maltodextrin), polyols (including mannitol, xylitol, and sorbitol), cyclodextrins and the like.

Suitable wetting agents for use in the solid dosage forms described herein include, for example, oleic acid, glyceryl monostearate, sorbitan monooleate, sorbitan monolaurate, triethanolamine oleate, polyoxyethylene sorbitan monooleate, polyoxyethylene sorbitan monolaurate, quaternary ammonium compounds (e.g., Polyquat 10^{®}), sodium oleate, sodium lauryl sulfate, magnesium stearate, sodium docusate, triacetin, vitamin E TPGS and the like.

Suitable surfactants for use in the solid dosage forms described herein include, for example, sodium lauryl sulfate, sorbitan monooleate, polyoxyethylene sorbitan monooleate, polysorbates, polaxomers, bile salts, glyceryl monostearate, copolymers of ethylene oxide and propylene oxide, e.g., Pluronic^{®} (BASF), and the like.

Suitable suspending agents for use in the solid dosage forms described here include, but are not limited to, polyvinylpyrrolidone, e.g., polyvinylpyrrolidone K12, polyvinylpyrrolidone K17, polyvinylpyrrolidone K25, or polyvinylpyrrolidone K30, polyethylene glycol, wherein e.g., the polyethylene glycol has a molecular weight of about 300 to about 6000, or about 3350 to about 4000, or about 5400 to about 7000, vinyl pyrrolidone/vinyl acetate copolymer (S630), sodium carboxymethylcellulose, methylcellulose, hydroxy-propylmethylcellulose, polysorbate-80, hydroxyethylcellulose, sodium alginate, gums, such as, e.g., gum tragacanth and gum acacia, guar gum, xanthans, including xanthan gum, sugars, cellulosics, such as, e.g., sodium carboxymethylcellulose, methylcellulose, sodium carboxymethylcellulose, hydroxypropylmethylcellulose, hydroxyethylcellulose, polysorbate-80, sodium alginate, polyethoxylated sorbitan monolaurate, polyethoxylated sorbitan monolaurate, povidone and the like.

Suitable antioxidants for use in the solid dosage forms described herein include, for example, e.g., butylated hydroxytoluene (BHT), sodium ascorbate, and tocopherol.

There is considerable overlap between additives used in the solid dosage forms described herein. In certain instances, the above-listed additives should be taken as merely exemplary, and not limiting, of the types of additives that can be included in solid dosage forms of the pharmaceutical compositions described herein.

In other cases, one or more layers of the pharmaceutical formulation are plasticized. Illustratively, a plasticizer is generally a high boiling point solid or liquid. In some instances, suitable plasticizers are added from about 0.01% to about 50% by weight (w/w) of the coating composition. Plasticizers include, but are not limited to, diethyl phthalate, citrate esters, polyethylene glycol, glycerol, acetylated glycerides, triacetin, polypropylene glycol, polyethylene glycol, triethyl citrate, dibutyl sebacate, stearic acid, stearol, stearate, and castor oil.

Compressed tablets are solid dosage forms prepared by compacting the bulk blend of the formulations described above. In various cases, compressed tablets which are designed to dissolve in the mouth will include one or more flavoring agents. In other cases, the compressed tablets will include a film surrounding the final compressed tablet. In some cases, the film coating provides a delayed release of any combination of anticancer therapy and two BER pathway inhibitors (such as methoxyamine and a PARP inhibitor) as described herein from the formulation. In other cases, the film coating aids in patient compliance (e.g., Opadry^{®} coatings or sugar coating). Film coatings including Opadry^{®} typically range from about 1% to about 3% of the tablet weight. In other cases, the compressed tablets include one or more excipients.

In some instances, a capsule is prepared, for example, by placing the bulk blend of the formulation of the compound described above, inside of a capsule. In some cases, the formulations (non-aqueous suspensions and solutions) are placed in a soft gelatin capsule. In other cases, the formulations are placed in standard gelatin capsules or non-gelatin capsules such as capsules comprising HPMC. In other cases, the formulation is placed in a sprinkle capsule, wherein the capsule is swallowed whole or the capsule is opened and the contents sprinkled on food prior to eating. In some cases, the therapeutic dose is split into multiple (e.g., two, three, or four) capsules. In some cases, the entire dose of the formulation is delivered in a capsule form.

In various cases, the particles of any combination of anticancer therapy and two BER pathway inhibitors (such as methoxyamine and a PARP inhibitor) as described herein and one or more excipients are dry blended and compressed into a mass, such as a tablet, having a hardness sufficient to provide a pharmaceutical composition that substantially disintegrates within less than about 30 minutes, less than about 35 minutes, less than about 40 minutes, less than about 45 minutes, less than about 50 minutes, less than about 55 minutes, or less than about 60 minutes, after oral administration, thereby releasing the formulation into the gastrointestinal fluid.

In another aspect, dosage forms include microencapsulated formulations. In some cases, one or more other compatible materials are present in the microencapsulation material. Exemplary materials include, but are not limited to, pH modifiers, erosion facilitators, anti-foaming agents, antioxidants, flavoring agents, and carrier materials such as binders, suspending agents, disintegration agents, filling agents, surfactants, solubilizers, stabilizers, lubricants, wetting agents, and diluents.

Materials useful for the microencapsulation described herein include materials compatible with compounds described herein, which sufficiently isolate the compound from other non-compatible excipients. Materials compatible with compounds described herein are those that delay the release of any combination of anticancer therapy and two BER pathway inhibitors (such as methoxyamine and a PARP inhibitor) as described herein *in vivo*.

Exemplary microencapsulation materials useful for delaying the release of the formulations including compounds described herein, include, but are not limited to, hydroxypropyl cellulose ethers (HPC) such as Klucel^{®} or Nisso HPC, low-substituted hydroxypropyl cellulose ethers (L-HPC), hydroxypropyl methyl cellulose ethers (HPMC) such as Seppifilm-LC, Pharmacoat^{®}, Metolose SR, Methocel^{®}-E, Opadry YS, PrimaFlo, Benecel MP824, and Benecel MP843, methylcellulose polymers such as Methocel^{®}-A, hydroxypropylmethylcellulose acetate stearate Aqoat (HF-LS, HF-LG,HF-MS) and Metolose^{®}, Ethylcelluloses (EC) and mixtures thereof such as E461, Ethocel^{®}, Aqualon^{®}-EC, Surelease^{®}, Polyvinyl alcohol (PVA) such as Opadry AMB, hydroxyethylcelluloses such as Natrosol^{®}, carboxymethylcelluloses and salts of carboxymethylcelluloses (CMC) such as Aqualon^{®}-CMC, polyvinyl alcohol and polyethylene glycol co-polymers such as Kollicoat IR^{®}, monoglycerides (Myverol), triglycerides (KLX), polyethylene glycols, modified food starch, acrylic polymers and mixtures of acrylic polymers with cellulose ethers such as Eudragit^{®} EPO, Eudragit^{®} L30D-55, Eudragit^{®} FS 30D Eudragit^{®} L100-55, Eudragit^{®} L100, Eudragit^{®} S100, Eudragit^{®} RD100, Eudragit^{®} E100, Eudragit^{®} L12.5, Eudragit^{®} S12.5, Eudragit^{®} NE30D, and Eudragit^{®} NE 40D, cellulose acetate phthalate, sepifilms such as mixtures of HPMC and stearic acid, cyclodextrins, and mixtures of these materials.

In still other cases, plasticizers such as polyethylene glycols, e.g., PEG 300, PEG 400, PEG 600, PEG 1450, PEG 3350, and PEG 800, stearic acid, propylene glycol, oleic acid, and triacetin are incorporated into the microencapsulation material. In other cases, the microencapsulating material useful for delaying the release of the pharmaceutical compositions is from the USP or the National Formulary (NF). In yet other cases, the microencapsulation material is Klucel. In still other cases, the microencapsulation material is methocel.

In some instances, microencapsulated compounds described herein are formulated by methods that include, e.g., spray drying processes, spinning disk-solvent processes, hot melt processes, spray chilling methods, fluidized bed, electrostatic deposition, centrifugal extrusion, rotational suspension separation, polymerization at liquid-gas or solid-gas interface, pressure extrusion, or spraying solvent extraction bath. In addition to these, several chemical techniques, e.g., complex coacervation, solvent evaporation, polymer-polymer incompatibility, interfacial polymerization in liquid media, in situ polymerization, in-liquid drying, and desolvation in liquid media could also be used. In certain instances, other methods such as roller compaction, extrusion/spheronization, coacervation, or nanoparticle coating are also used.

In still other cases, effervescent powders are also prepared in accordance with the present disclosure. Effervescent salts have been used to disperse medicines in water for oral administration. Effervescent salts are granules or coarse powders containing a medicinal agent in a dry mixture, usually composed of sodium bicarbonate, citric acid and/or tartaric acid. When such salts are added to water, the acids and the base react to liberate carbon dioxide gas, thereby causing "effervescence." Examples of effervescent salts include, e.g., the following ingredients: sodium bicarbonate or a mixture of sodium bicarbonate and sodium carbonate, citric acid and/or tartaric acid. Any acid-base combination that results in the liberation of carbon dioxide can be used in place of the combination of sodium bicarbonate and citric and tartaric acids, as long as the ingredients were suitable for pharmaceutical use and result in a pH of about 6.0 or higher.

In other cases, the formulations described herein, which include any combination of anticancer therapy with two BER pathway inhibitors (such as methoxyamine and a PARP inhibitor) described herein, are solid dispersions. Methods of producing such solid dispersions include, but are not limited to, for example, U.S. Pat. Nos. 4,343,789, 5,340,591, 5,456,923, 5,700,485, 5,723,269, and U.S. patent publication no. 2004/0013734. In still other cases, the formulations described herein are solid solutions. Solid solutions incorporate a substance together with the active agent and other excipients such that heating the mixture results in dissolution of the drug and the resulting composition is then cooled to provide a solid blend which in some instances is further formulated or directly added to a capsule or compressed into a tablet. Methods of producing such solid solutions include, but are not limited to, for example, U.S. Pat. Nos. 4,151,273, 5,281,420, and 6,083,518.

In certain cases, the pharmaceutical solid oral dosage forms including formulations described herein, which include any combination of anticancer therapy and two BER pathway inhibitors (such as methoxyamine and a PARP inhibitor) as described herein, are further formulated to provide a controlled release of any combination of anticancer therapy and two BER pathway inhibitors (such as methoxyamine and a PARP inhibitor) as described herein. Controlled release refers to the release of the compounds described herein from a dosage form in which it is incorporated according to a desired profile over an extended period of time. Controlled release profiles include, for example, sustained release, prolonged release, pulsatile release, and delayed release profiles. In contrast to immediate release compositions, controlled release compositions allow delivery of an agent to a subject over an extended period of time according to a predetermined profile. In some instances, such release rates provide therapeutically effective levels of agent for an extended period of time and thereby provide a longer period of pharmacologic response while minimizing side effects as compared to conventional rapid release dosage forms. Such longer periods of response provide for many inherent benefits that are not achieved with the corresponding short acting, immediate release preparations.

In some cases, the solid dosage forms described herein is formulated as enteric coated delayed release oral dosage forms, i.e., as an oral dosage form of a pharmaceutical composition as described herein which utilizes an enteric coating to affect release in the small intestine of the gastrointestinal tract. In some instances, the enteric coated dosage form is a compressed or molded or extruded tablet/mold (coated or uncoated) containing granules, powder, pellets, beads or particles of the active ingredient and/or other composition components, which are themselves coated or uncoated. In certain instances, the enteric coated oral dosage form is also a capsule (coated or uncoated) containing pellets, beads or granules of the solid carrier or the composition, which are themselves coated or uncoated.

The term "delayed release" as used herein refers to the delivery so that the release is accomplished at some generally predictable location in the intestinal tract more distal to that which would have been accomplished if there had been no delayed release alterations. In some cases the method for delay of release is coating. Any coatings should be applied to a sufficient thickness such that the entire coating does not dissolve in the gastrointestinal fluids at pH below about 5, but does dissolve at pH about 5 and above.

The performance of acrylic polymers (primarily their solubility in biological fluids) varies based on the degree and type of substitution. Examples of suitable acrylic polymers include methacrylic acid copolymers and ammonium methacrylate copolymers. The Eudragit^{®} series E, L, S, RL, RS and NE (Rohm Pharma) are available as solubilized in organic solvent, aqueous dispersion, or dry powders. The Eudragit^{®} series RL, NE, and RS are insoluble in the gastrointestinal tract but are permeable and are used primarily for colonic targeting. The Eudragit® series E dissolve in the stomach. The Eudragit® series L, L-30D and S are insoluble in stomach and dissolve in the intestine;

Examples of suitable cellulose derivatives are: ethyl cellulose; reaction mixtures of partial acetate esters of cellulose with phthalic anhydride. In some instances, the performance varies based on the degree and type of substitution. Cellulose acetate phthalate (CAP) dissolves in pH >6. Aquateric (FMC) is an aqueous based system and is a spray dried CAP pseudolatex with particles <1 µm In certain instances, other components in Aquateric include pluronics, Tweens, and acetylated monoglycerides. Other suitable cellulose derivatives include: cellulose acetate trimellitate (Eastman); methylcellulose (Pharmacoat, Methocel); hydroxypropylmethyl cellulose phthalate (HPMCP); hydroxypropylmethyl cellulose succinate (HPMCS); and hydroxypropylmethylcellulose acetate succinate (e.g., AQOAT (Shin Etsu)). In some instances, the performance varies based on the degree and type of substitution. For example, HPMCP such as, HP-50, HP-55, HP-55S, HP-55F grades are suitable. In certain instances, the performance varies based on the degree and type of substitution. For example, suitable grades of hydroxypropylmethylcellulose acetate succinate include, but are not limited to, AS-LG (LF), which dissolves at pH 5, AS-MG (MF), which dissolves at pH 5.5, and AS-HG (HF), which dissolves at higher pH. These polymers are offered as granules, or as fine powders for aqueous dispersions;

Poly Vinyl Acetate Phthalate (PVAP) dissolves in pH >5, and it is much less permeable to water vapor and gastric fluids.

In some cases, the coating contains a plasticizer and possibly other coating excipients such as colorants, talc, and/or magnesium stearate. Suitable plasticizers include triethyl citrate (Citroflex 2), triacetin (glyceryl triacetate), acetyl triethyl citrate (Citroflec A2), Carbowax 400 (polyethylene glycol 400), diethyl phthalate, tributyl citrate, acetylated monoglycerides, glycerol, fatty acid esters, propylene glycol, and dibutyl phthalate. In particular, anionic carboxylic acrylic polymers usually will contain 10-25% by weight of a plasticizer, especially dibutyl phthalate, polyethylene glycol, triethyl citrate and triacetin. Conventional coating techniques such as spray or pan coating are employed to apply coatings. The coating thickness must be sufficient to ensure that the oral dosage form remains intact until the desired site of topical delivery in the intestinal tract is reached.

In some instances, colorants, detackifiers, surfactants, antifoaming agents, lubricants (e.g., carnuba wax or PEG) are added to the coatings besides plasticizers to solubilize or disperse the coating material, and to improve coating performance and the coated product.

In other cases, the formulations described herein, which include any combination of anticancer therapy with two BER pathway inhibitors (such as methoxyamine and a PARP inhibitor) as described herein, are delivered using a pulsatile dosage form. A pulsatile dosage form is capable of providing one or more immediate release pulses at predetermined time points after a controlled lag time or at specific sites. In some instances, pulsatile dosage forms are administered using a variety of pulsatile formulations including, but are not limited to, those described in U.S. Pat. Nos. 5,011,692; 5,017,381; 5,229,135; 5,840,329; 4,871,549; 5,260,068; 5,260,069; 5,508,040; 5,567,441 and 5,837,284.

Many other types of controlled release systems are suitable for use with the formulations described herein. Examples of such delivery systems include, e.g., polymer-based systems, such as polylactic and polyglycolic acid, polyanhydrides and polycaprolactone; porous matrices, nonpolymer-based systems that are lipids, including sterols, such as cholesterol, cholesterol esters and fatty acids, or neutral fats, such as mono-, di- and triglycerides; hydrogel release systems; silastic systems; peptide-based systems; wax coatings, bioerodible dosage forms, compressed tablets using conventional binders and the like. See, e.g., Liberman et al., Pharmaceutical Dosage Forms, 2 Ed., Vol. 1, pp. 209-214 (1990); Singh et al., Encyclopedic of Pharmaceutical Technology, 2nd Ed., pp. 751-753 (2002); U.S. Pat. Nos. 4,327,725; 4,624,848; 4,968,509; 5,461,140; 5,456,923; 5,516,527; 5,622,721; 5,686,105; 5,700,410; 5,977,175; 6,465,014; and 6,932,983.

In some cases, pharmaceutical formulations are provided that include particles of the compounds described herein, e.g. any combination of anticancer therapy with two BER pathway inhibitors (such as methoxyamine and a PARP inhibitor) as described herein, and at least one dispersing agent or suspending agent for oral administration to a subject. In some instances, the formulations are a powder and/or granules for suspension, and upon admixture with water, a substantially uniform suspension is obtained.

In some instances, liquid formulation dosage forms for oral administration are aqueous suspensions selected from the group including, but not limited to, pharmaceutically acceptable aqueous oral dispersions, emulsions, solutions, elixirs, gels, and syrups. See, e.g., Singh et al., Encyclopedic of Pharmaceutical Technology, 2nd Ed., pp. 754-757 (2002).

In certain instances, aqueous suspensions and dispersions described herein remain in a homogenous state, as defined in The USP Pharmacists' Pharmacopeia (2005 edition, chapter 905), for at least 4 hours. The homogeneity should be determined by a sampling method consistent with regard to determining homogeneity of the entire composition. In one case, an aqueous suspension is re-suspended into a homogenous suspension by physical agitation lasting less than 1 minute. In another case, an aqueous suspension is re-suspended into a homogenous suspension by physical agitation lasting less than 45 seconds. In yet another case, an aqueous suspension is re-suspended into a homogenous suspension by physical agitation lasting less than 30 seconds. In still another case, no agitation is necessary to maintain a homogeneous aqueous dispersion.

In certain cases, the pharmaceutical compositions described herein include sweetening agents such as, but not limited to, acacia syrup, acesulfame K, alitame, anise, apple, aspartame, banana, Bavarian cream, berry, black currant, butterscotch, calcium citrate, camphor, caramel, cherry, cherry cream, chocolate, cinnamon, bubble gum, citrus, citrus punch, citrus cream, cotton candy, cocoa, cola, cool cherry, cool citrus, cyclamate, cylamate, dextrose, eucalyptus, eugenol, fructose, fruit punch, ginger, glycyrrhetinate, glycyrrhiza (licorice) syrup, grape, grapefruit, honey, isomalt, lemon, lime, lemon cream, monoammonium glyrrhizinate (MagnaSweet^{®}), maltol, mannitol, maple, marshmallow, menthol, mint cream, mixed berry, neohesperidine DC, neotame, orange, pear, peach, peppermint, peppermint cream, Prosweet^{®} Powder, raspberry, root beer, rum, saccharin, safrole, sorbitol, spearmint, spearmint cream, strawberry, strawberry cream, stevia, sucralose, sucrose, sodium saccharin, saccharin, aspartame, acesulfame potassium, mannitol, talin, sucralose, sorbitol, swiss cream, tagatose, tangerine, thaumatin, tutti fruitti, vanilla, walnut, watermelon, wild cherry, wintergreen, xylitol, or any combination of these flavoring ingredients, e.g., anise-menthol, cherry-anise, cinnamon-orange, cherry-cinnamon, chocolate-mint, honey-lemon, lemon-lime, lemon-mint, menthol-eucalyptus, orange-cream, vanilla-mint, and mixtures thereof.

In some cases, the pharmaceutical formulations described herein are self-emulsifying drug delivery systems (SEDDS). Emulsions are dispersions of one immiscible phase in another, usually in the form of droplets. Generally, emulsions are created by vigorous mechanical dispersion. SEDDS, as opposed to emulsions or microemulsions, spontaneously form emulsions when added to an excess of water without any external mechanical dispersion or agitation. An advantage of SEDDS is that only gentle mixing is required to distribute the droplets throughout the solution. In certain instances, water or the aqueous phase are added just prior to administration, which ensures stability of an unstable or hydrophobic active ingredient. In some instances, the SEDDS provides an effective delivery system for oral and parenteral delivery of hydrophobic active ingredients. In certain instances, SEDDS provides improvements in the bioavailability of hydrophobic active ingredients. Methods of producing self-emulsifying dosage forms include, but are not limited to, for example, U.S. Pat. Nos. 5,858,401, 6,667,048, and 6,960,563.

There is overlap between the above-listed additives used in the aqueous dispersions or suspensions described herein, since a given additive is often classified differently by different practitioners in the field, or is commonly used for any of several different functions. Thus, the above-listed additives should be taken as merely exemplary, and not limiting, of the types of additives that can be included in formulations described herein.

Potential excipients for intranasal formulations include, for example, U.S. Pat. Nos. 4,476,116, 5,116,817 and 6,391,452. Formulations solutions in saline, employing benzyl alcohol or other suitable preservatives, fluorocarbons, and/or other solubilizing or dispersing agents. See, for example, Ansel, H. C. et al., Pharmaceutical Dosage Forms and Drug Delivery Systems, Sixth Ed. (1995). Preferably these compositions and formulations are prepared with suitable nontoxic pharmaceutically acceptable ingredients. The choice of suitable carriers is highly dependent upon the exact nature of the nasal dosage form desired, e.g., solutions, suspensions, ointments, or gels. Nasal dosage forms generally contain large amounts of water in addition to the active ingredient. In some instances, minor amounts of other ingredients such as pH adjusters, emulsifiers or dispersing agents, preservatives, surfactants, gelling agents, or buffering and other stabilizing and solubilizing agents are also present. Preferably, the nasal dosage form should be isotonic with nasal secretions.

In some cases, buccal formulations that include compounds described herein are administered using a variety of formulations which include, but are not limited to, U.S. Pat. Nos. 4,229,447, 4,596,795, 4,755,386, and 5,739,136. In some instances, the buccal dosage forms described herein further include a bioerodible (hydrolysable) polymeric carrier that also serves to adhere the dosage form to the buccal mucosa. The buccal dosage form is fabricated so as to erode gradually over a predetermined time period, wherein the delivery of the compound is provided essentially throughout. Buccal drug delivery avoids the disadvantages encountered with oral drug administration, e.g., slow absorption, degradation of the active agent by fluids present in the gastrointestinal tract and/or first-pass inactivation in the liver. In certain instances, with regard to the bioerodible (hydrolysable) polymeric carrier, virtually any such carrier can be used, so long as the desired drug release profile is not compromised, and the carrier is compatible with the compounds described herein, and any other components that are present in the buccal dosage unit. Generally, the polymeric carrier comprises hydrophilic (water-soluble and water-swellable) polymers that adhere to the wet surface of the buccal mucosa. Examples of polymeric carriers useful herein include acrylic acid polymers and co, e.g., those known as "carbomers" (Carbopol^{®}, which is for example obtained from B.F. Goodrich, is one such polymer). In some instances, other components which are also incorporated into the buccal dosage forms described herein include, but are not limited to, disintegrants, diluents, binders, lubricants, flavoring, colorants, preservatives, and the like. In some instances, for buccal or sublingual administration, the compositions take the form of tablets, lozenges, or gels formulated in a conventional manner.

In some cases, transdermal formulations described herein are administered using a variety of devices including but not limited to, U.S. Pat. Nos. 3,598,122, 3,598,123, 3,710,795, 3,731,683, 3,742,951, 3,814,097, 3,921,636, 3,972,995, 3,993,072, 3,993,073, 3,996,934, 4,031,894, 4,060,084, 4,069,307, 4,077,407, 4,201,211, 4,230,105, 4,292,299, 4,292,303, 5,336,168, 5,665,378, 5,837,280, 5,869,090, 6,923,983, 6,929,801 and 6,946,144.

In certain instances, the transdermal dosage forms described herein incorporates certain pharmaceutically acceptable excipients which are conventional in the art. In one case, the transdermal formulations described herein include at least three components: (1) a formulation of any combination of anticancer therapy with two BER pathway inhibitors (such as methoxyamine and a PARP inhibitor) as described herein; (2) a penetration enhancer; and (3) an aqueous adjuvant. In some instances, transdermal formulations include additional components such as, but not limited to, gelling agents, creams and ointment bases, and the like. In some cases, the transdermal formulation further includes a woven or non-woven backing material to enhance absorption and prevent the removal of the transdermal formulation from the skin. In other cases, the transdermal formulations described herein maintain a saturated or supersaturated state to promote diffusion into the skin.

In some instances, formulations suitable for transdermal administration of compounds described herein employ transdermal delivery devices and transdermal delivery patches and are lipophilic emulsions or buffered, aqueous solutions, dissolved and/or dispersed in a polymer or an adhesive. In certain instances, such patches are constructed for continuous, pulsatile, or on demand delivery of pharmaceutical agents. In some instances, transdermal delivery of the compounds described herein is accomplished by means of iontophoretic patches and the like. In certain instances, transdermal patches provide controlled delivery of the compounds described herein. In some instances, the rate of absorption is slowed by using rate-controlling membranes or by trapping the compound within a polymer matrix or gel. In certain instances, absorption enhancers are used to increase absorption. In some instances, an absorption enhancer or carrier includes absorbable pharmaceutically acceptable solvents to assist passage through the skin. For example, transdermal devices are in the form of a bandage comprising a backing member, a reservoir containing the compound optionally with carriers, optionally a rate controlling barrier to deliver the compound to the skin of the host at a controlled and predetermined rate over a prolonged period of time, and means to secure the device to the skin.

In some cases, formulations suitable for intramuscular, subcutaneous, or intravenous injection include physiologically acceptable sterile aqueous or non-aqueous solutions, dispersions, suspensions or emulsions, and sterile powders for reconstitution into sterile injectable solutions or dispersions. Examples of suitable aqueous and non-aqueous carriers, diluents, solvents, or vehicles including water, ethanol, polyols (propyleneglycol, polyethylene-glycol, glycerol, cremophor and the like), suitable mixtures thereof, vegetable oils (such as olive oil) and injectable organic esters such as ethyl oleate. In some instances, proper fluidity is maintained, for example, by the use of a coating such as lecithin, by the maintenance of the required particle size in the case of dispersions, and by the use of surfactants. In some instances, formulations suitable for subcutaneous injection also contain additives such as preserving, wetting, emulsifying, and dispensing agents. In certain instances, prevention of the growth of microorganisms is ensured by various antibacterial and antifungal agents, such as parabens, chlorobutanol, phenol, sorbic acid, and the like. In specific instances, it is also desirable to include isotonic agents, such as sugars, sodium chloride, and the like. In some instances, prolonged absorption of the injectable pharmaceutical form is brought about by the use of agents delaying absorption, such as aluminum monostearate and gelatin.

In certain cases, for intravenous injections compounds described herein are formulated in aqueous solutions, preferably in physiologically compatible buffers such as Hank's solution, Ringer's solution, or physiological saline buffer. For transmucosal administration, penetrants appropriate to the barrier to be permeated are used in the formulation. Such penetrants are generally recognized in the field. In some instances, for other parenteral injections, appropriate formulations include aqueous or nonaqueous solutions, preferably with physiologically compatible buffers or excipients. Such excipients are generally recognized in the field.

In certain cases, parenteral injections involve bolus injection or continuous infusion. In some instances, formulations for injection are presented in unit dosage form, e.g., in ampoules or in multi-dose containers, with an added preservative. In some instances, the pharmaceutical composition described herein is in a form suitable for parenteral injection as a sterile suspensions, solutions or emulsions in oily or aqueous vehicles, and contains formulatory agents such as suspending, stabilizing and/or dispersing agents. Pharmaceutical formulations for parenteral administration include aqueous solutions of the active compounds in water-soluble form. In certain instances, suspensions of the active compounds are prepared as appropriate oily injection suspensions. Suitable lipophilic solvents or vehicles include fatty oils such as sesame oil, or synthetic fatty acid esters, such as ethyl oleate or triglycerides, or liposomes. In some instances, aqueous injection suspensions contain substances which increase the viscosity of the suspension, such as sodium carboxymethyl cellulose, sorbitol, or dextran. In certain instances, the suspension also contains suitable stabilizers or agents which increase the solubility of the compounds to allow for the preparation of highly concentrated solutions. In other instances, the active ingredient is in powder form for constitution with a suitable vehicle, e.g., sterile pyrogen-free water, before use.

In certain cases, delivery systems for pharmaceutical compounds are employed, such as, for example, liposomes and emulsions. In certain cases, compositions provided herein also include an mucoadhesive polymer, selected from among, for example, carboxymethylcellulose, carbomer (acrylic acid polymer), poly(methylmethacrylate), polyacrylamide, polycarbophil, acrylic acid/butyl acrylate copolymer, sodium alginate and dextran.

In some cases, any combination of anticancer therapy with two BER pathway inhibitors (such as methoxyamine and a PARP inhibitor) as described herein is administered topically and is formulated into a variety of topically administrable compositions, such as solutions, suspensions, lotions, gels, pastes, medicated sticks, balms, creams or ointments. In some instances, such pharmaceutical compounds contain solubilizers, stabilizers, tonicity enhancing agents, buffers and preservatives.

In certain cases, the compounds described herein are formulated in rectal compositions such as enemas, rectal gels, rectal foams, rectal aerosols, suppositories, jelly suppositories, or retention enemas, containing conventional suppository bases such as cocoa butter or other glycerides, as well as synthetic polymers such as polyvinylpyrrolidone, PEG, and the like. In suppository forms of the compositions, a low-melting wax such as, but not limited to, a mixture of fatty acid glycerides, optionally in combination with cocoa butter is first melted.

In some cases, the pharmaceutical composition described herein are be in unit dosage forms suitable for single administration of precise dosages. In unit dosage form, the formulation is divided into unit doses containing appropriate quantities of one or more compound. In some instances, the unit dosage is in the form of a package containing discrete quantities of the formulation. Non-limiting examples are packaged tablets or capsules, and powders in vials or ampoules. In some instances, aqueous suspension compositions are packaged in single-dose non-reclosable containers. In other instances, multiple-dose reclosable containers are used, in which case it is typical to include a preservative in the composition. In some instances, by way of example only, formulations for parenteral injection are presented in unit dosage form, which include, but are not limited to ampoules, or in multi-dose containers, with an added preservative.

### Biological Activity

In certain cases, administering a combination of an anticancer agent (such as TMZ) with two BER pathway inhibitors (such as methoxyamine and a PARP inhibitor) potentiates the cytotoxicity in cancer cell lines compared to administering the anticancer agent alone by about 4- to about 10-fold. In some cases, administering a combination of an anticancer agent (such as TMZ) with two BER pathway inhibitors (such as methoxyamine and a PARP inhibitor) potentiates the cytotoxicity in cancer cell lines compared to administering the anticancer agent alone by about 2-fold, about 3-fold, about 4-fold, about 5-fold, about 6-fold, about 7-fold, about 8-fold, about 9-fold, about 10-fold, about 12-fold, about 15-fold, about 20-fold, or about 25-fold. In certain cases, administering a combination of an anticancer agent (such as TMZ) with two BER pathway inhibitors (such as methoxyamine and a PARP inhibitor) potentiates the cytotoxicity in cancer cell lines compared to administering the anticancer agent alone by more than 2-fold, more than 3-fold, more than 4-fold, more than 5-fold, more than 6-fold, more than 7-fold, more than 8-fold, more than 9-fold, more than 10-fold, more than 12-fold, more than 15-fold, more than 20-fold, or more than 25-fold. In some cases, administering a combination of an anticancer agent (such as TMZ) with two BER pathway inhibitors (such as methoxyamine and a PARP inhibitor) potentiates the cytotoxicity in cancer cell lines compared to administering the anticancer agent alone by less than 2-fold, less than 3-fold, less than 4-fold, less than 5-fold, less than 6-fold, less than 7-fold, less than 8-fold, less than 9-fold, less than 10-fold, less than 12-fold, less than 15-fold, less than 20-fold, or less than 25-fold. In certain cases, administering a combination of an anticancer agent (such as TMZ) with two BER pathway inhibitors (such as methoxyamine and a PARP inhibitor) potentiates the cytotoxicity in cancer cell lines compared to administering the anticancer agent alone by more than 2-fold and less than 10-fold. In some cases, administering a combination of an anticancer agent (such as TMZ) with two BER pathway inhibitors (such as methoxyamine and a PARP inhibitor) potentiates the cytotoxicity in cancer cell lines compared to administering the anticancer agent alone by more than 10-fold and less than 25-fold. In certain cases, administering a combination of an anticancer agent (such as TMZ) with two BER pathway inhibitors (such as methoxyamine and a PARP inhibitor) potentiates the cytotoxicity in cancer cell lines compared to administering the anticancer agent alone by more than 5-fold and less than 15-fold.

In certain cases, administering a combination of an anticancer agent (such as TMZ) with two BER pathway inhibitors (such as methoxyamine and a PARP inhibitor) reduces tumor volume compared to administering the anticancer agent alone by about 60% to 80%. In some cases, administering a combination of an anticancer agent (such as TMZ) with two BER pathway inhibitors (such as methoxyamine and a PARP inhibitor) reduces tumor volume compared to administering the anticancer agent alone by about 10%, about 20%, about 30%, about 40%, about 50%, about 60%, about 70%, about 80%, or about 90%. In some cases, administering a combination of an anticancer agent (such as TMZ) with two BER pathway inhibitors (such as methoxyamine and a PARP inhibitor) reduces tumor volume compared to administering the anticancer agent alone by more than 10%, more than 20%, more than 30%, more than 40%, more than 50%, more than 60%, more than 70%, more than 80%, or more than 90%. In some cases, administering a combination of an anticancer agent (such as TMZ) with two BER pathway inhibitors (such as methoxyamine and a PARP inhibitor) reduces tumor volume compared to administering the anticancer agent alone by less than 10%, less than 20%, less than 30%, less than 40%, less than 50%, less than 60%, less than 70%, less than 80%, or less than 90%. In certain cases, administering a combination of an anticancer agent (such as TMZ) with two BER pathway inhibitors (such as methoxyamine and a PARP inhibitor) reduces tumor volume compared to administering the anticancer agent alone by more than 50% and less than 90%. In some cases, administering a combination of an anticancer agent (such as TMZ) with two BER pathway inhibitors (such as methoxyamine and a PARP inhibitor) reduces tumor volume compared to administering the anticancer agent alone by more than 10% and less than 50%. In certain cases, administering a combination of an anticancer agent (such as TMZ) with two BER pathway inhibitors (such as methoxyamine and a PARP inhibitor) reduces tumor volume compared to administering the anticancer agent alone by more than 40% and less than 80%.

### EXAMPLES

The application may be better understood by reference to the following non-limiting examples, which are provided as exemplary cases of the application. The following examples are presented in order to more fully illustrate cases of the invention and should in no way be construed, however, as limiting the broad scope of the application. While certain cases of the present application have been shown and described herein, it will be obvious that such cases are provided by way of example only. Numerous variations, changes, and substitutions may occur to those skilled in the art without departing from the invention; it should be understood that various alternatives to the cases described herein may be employed in practicing the methods described herein.

The starting materials and reagents used for the processes, methods, and compositions described herein are synthesized or are obtained from commercial sources. A375, WM9, and WM164 cells were obtained from commercial sources. All cell lines were cultured in appropriate growth media.

### EXAMPLE 1: Colony Survival Assay

Cells (2000/dish) were plated, adhered for 18 h, and treated with TMZ plus or minus modifiers, such as MX and ABT-888, according to experimental protocol. After treatment, the cells were washed and fresh medium was added. The cells were grown for a further 7 days prior to staining with methylene blue for determination of colonies containing more than 50 cells. Comparisons of drug-induced cytotoxicity consisted of a calculation of the dose modification factor (DMF), defined as the ratio of the IC50 of TMZ in the absence of indicated modifier(s) that that in the presence of indicated modifier(s), *i.e.,* DMF = IC50 for TMZ alone / IC50 for TMZ plus modifier(s). The DMF indicates the degree of potentiation of cytotoxic agents by a modulator.

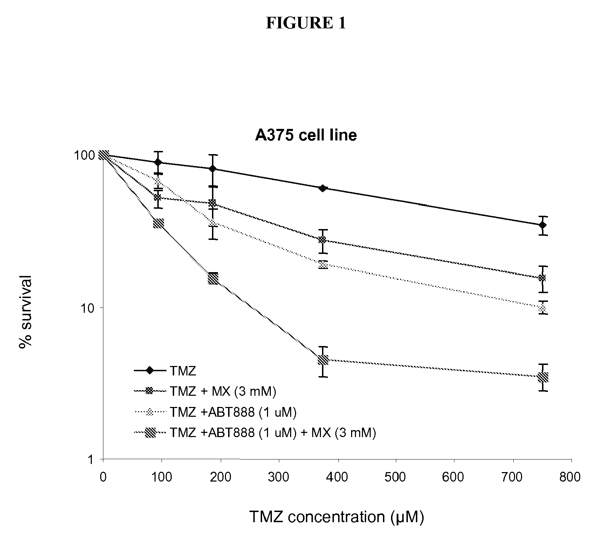

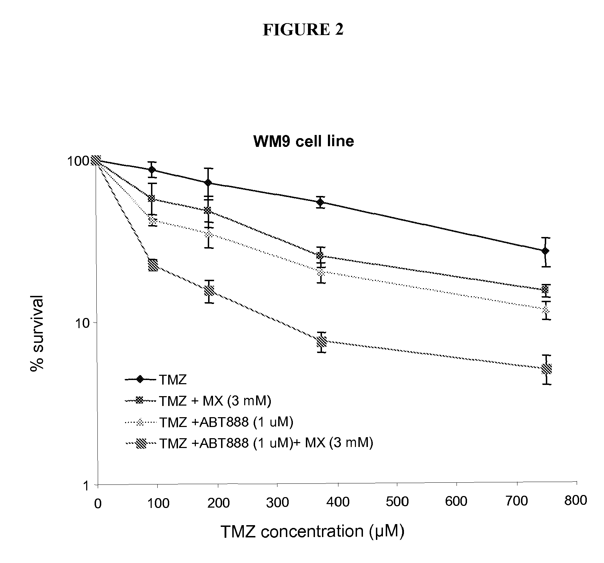

In some instances, the combination of an alkylating agent with a PARP inhibitor more effectively inhibited cell viability and induced apoptosis. The combination of the PARP inhibitor ABT-888 with TMZ more effectively inhibited cell viability and induced apoptosis in A375 and WM9 cell lines as compared to TMZ alone, as illustrated in **Figures 1-2****.** The combination of the AP site binder methoxyamine (MX) with TMZ more effectively inhibited cell viability and induced apoptosis in A375 and WM9 cell lines as compared to TMZ alone, as illustrated in **Figures 1-2****.** The combination of the AP site binder MX and the PARP inhibitor ABT-888 at a concentration of 5 µM with the alkylating agent TMZ efficiently potentiated the cytotoxicity by 8-10 fold in A375 and WM9 melanoma cell lines, and 4-fold in WM164 cells as compared to TMZ alone (**Figures 1-****2**).

In certain instances, the combination of the alkylating agent TMZ with one individual BER pathway inhibitor, e.g., the AP site binder MX on its own or the PARP inhibitor ABT-888 on its own, did not more effectively inhibit cell viability and induce apoptosis in WM164 cells as compared to TMZ alone. Without being bound by any particular theory, the resistance in WM164 to potentiation is related to a deficiency in methypurine-DNA glycosylase, which is responsible for removing TMZ-induced methylated DNA adducts (N7mG and N3mA) and producing toxic AP sites. But the combination of the alkylating agent TMZ with AP site binder MX and the PARP inhibitor ABT-888 efficiently potentiated the cytotoxicity in WM164 cells by 4 fold as compared to TMZ alone. In some instances, cytotoxicity is correlated with the induction of DNA single and double stranded breaks as assayed by comet assay and induction of γ-H2AX.

### EXAMPLE 2: Melanoma Xenografts

Tumor cells (5 x 10⁶) are injected into the bilateral flanks of female thymic nude mice (6-8 weeks of age). The tumors are measured with calipers using the formula: *V* = *L* (mm) x *I*² (mm)/2, where *V* is the volume, *L* is the largest diameter, and *I* is the perpendicular diameter of the tumor. When the volume of the tumor nodules reaches 100-150 mm³, mice are randomly assigned to control or treatment groups (6-9 mice/group).

Therapeutic treatment with a combination of the AP site binder MX and the PARP inhibitor ABT-888 with the alkylating agents TMZ was initiated when tumor xenografts (WM9) in nude mice reach 100 mm³ in volume and treatment was continued for 5 days. In specific instances, the tumor volume was measured for assessment of therapeutic effect. No significant differences in tumor growth were observed in mice treated with TMZ and in untreated mice. A 30-40% reduction in tumor volume at termination was found with a combination of TMZ with the PARP inhibitor ABT-888 or with a combination of TMZ with the AP site binder MX, when compared with the tumor reduction in TMZ only treated mice. An 80% reduction in tumor volume at termination was found with a combination of TMZ with the PARP inhibitor ABT-888 and the AP site binder MX, when compared with the tumor reduction in TMZ only treated mice. In some instances, the data indicate that combining the PARP inhibitor ABT-888 with MX and TMZ results in greater inhibition of BER and induces a synergistic cytotoxic effect.

### EXAMPLE 3: Method of Treatment - Antimetabolite Treatment Combined with Methoxyamine and a PARP Inhibitor

Human Clinical Trial of the Safety and Pharmacokinetics of Antimetabolite Treatment in Combination with Two BER Pathway Inhibitors in the Treatment of Cancer.

**Objective:** To determine the safety, tolerability, and pharmacokinetics of methoxyamine and the selective PARP inhibitor ABT-888 in combination with pemetrexed antimetabolite therapy in the treatment of subjects with advanced and metastatic solid cancers.

**Study Design:** This will be a Phase I, multi-center, open-label, non-randomized dose escalation study in cancer patients for the treatment of advanced or metastatic solid cancers. Patients present with advanced or metastatic solid cancer for which curative therapy is unavailable. Their ECOG (Eastern Conference Oncology Group) performance status is 0 or 1 and they have adequate organ function. Patients should not have had exposure to any BER pathway inhibitor study drug (e.g., PARP inhibitor and/or methoxyamine) prior to study entry. Patients must not have received treatment for their cancer within 4 weeks prior to the beginning of the trial. Treatments include the use of chemotherapy, immunotherapy, biologic therapy such as monoclonal antibodies, or investigational therapy. Patients must not display unresolved or unstable, serious toxicity from prior administration of another investigational drug and/or prior anticancer treatment or have had prior treatment with high-dose chemotherapy requiring stem cell rescue. The subject must not have a history of primary and secondary brain tumors. All subjects are evaluated for safety and all blood collections for pharmacokinetic analysis are collected as scheduled. All studies are performed with institutional ethics committee approval and patient consent.

**Phase I:** All patients are dosed with the methoxyamine and ABT-888 alone on days 1-4 of the initial two week cycle. Beginning with the second cycle, which is three weeks, the subjects are dosed in cohorts of 3-6 patients in combination with a standard dose of the antimetabolite pemetrexed (i.v. pemetrexed at doses of about 500 mg/kg), which is given on day one of the cycle. Patients also receive methoxyamine and ABT-888 (e.g., oral treatment with the selective PARP inhibitor ABT-888 at doses up to 40 mg bid, and oral solution treatment with the AP site binder methoxyamine at doses up to 100 mg/kg) daily during on days 1-4 of the treatment cycles. Doses of the selective PARP inhibitor ABT-888 and methoxyamine may be held or modified for toxicity. Treatment repeats every 21 days in the absence of unacceptable toxicity. Cohorts of patients receive fixed doses of the one BER pathway inhibitor (e.g., ABT-888) and escalating doses of the other BER pathway inhibitor (e.g., methoxyamine) until the maximum tolerated dose (MTD) for the combination is determined. The MTD is defined as the dose preceding that at which 2 of 3 or 2 of 6 patients experience dose-limiting toxicity. Dose limiting toxicities are determined in any suitable manner, e.g., according to the definitions and standards set by the National Cancer Institute (NCI) Common Terminology for Adverse Events (CTCAE) Version 3.0 (August 9, 2006).

Blood Sampling: Serial blood is drawn by direct vein puncture before and after administration of the methoxyamine and ABT-888. Venous blood samples (5 mL) for determination of serum concentrations are obtained at about 10 minutes prior to dosing and at specified times after dosing. Each serum sample is divided into two aliquots. All serum samples are stored at -20°C. Serum samples are shipped on dry ice.

Pharmacokinetics: Patients undergo plasma/serum sample collection for pharmacokinetic evaluation before beginning treatment and at specified days throughout the treatment cycle. Pharmacokinetic parameters are calculated by model independent methods on a Digital Equipment Corporation VAX 8600 computer system using the latest version of the BIOAVL software. The following pharmacokinetics parameters are determined: peak serum concentration (Cₘₐₓ); time to peak serum concentration (tₘₐₓ); area under the concentration-time curve (AUC) from time zero to the last blood sampling time (AUC₀₋₇₂) calculated with the use of the linear trapezoidal rule; and terminal elimination half-life (t_{1/2}), computed from the elimination rate constant. The elimination rate constant is estimated by linear regression of consecutive data points in the terminal linear region of the log-linear concentration-time plot. The mean, standard deviation (SD), and coefficient of variation (CV) of the pharmacokinetic parameters are calculated for each treatment. The ratio of the parameter means (preserved formulation/non-preserved formulation) is calculated.

### EXAMPLE 4: Method of Treatment - Alkylating Agent Treatment Combined with Methoxyamine and a PARP Inhibitor

Human Clinical Trial of the Safety and Pharmacokinetics of Antimetabolite Treatment in Combination with Two BER Pathway Inhibitors in the Treatment of Cancer.

**Objective:** To determine the safety, tolerability, and pharmacokinetics of methoxyamine and the selective PARP inhibitor ABT-888 in combination with temozolomide alkylating agent therapy in the treatment of subjects with advanced solid cancers.

**Study Design:** This will be a Phase I, multi-center, open-label, non-randomized dose escalation study in cancer patients for the treatment of advanced solid cancers. Patients present with advanced solid cancer and for whom curative therapy is unavailable. Their ECOG (Eastern Conference Oncology Group) performance status is 0-2 and their Karnofsky Performance Status (KPS) is greater than or equal to a score of 50. Patients should not have had exposure to any BER pathway inhibitor study drug (e.g., PARP inhibitor and/or methoxyamine) prior to study entry. Patients must not have received treatment for their cancer within 4 weeks prior to the beginning of the trial (6 weeks for mitomycin C and nitrosoureas). Treatments include the use of chemotherapy, immunotherapy, biologic therapy such as monoclonal antibodies, or investigational therapy. Patients must not display unresolved or unstable, serious toxicity from prior administration of another investigational drug and/or prior anticancer treatment or have had prior treatment with high-dose chemotherapy requiring stem cell rescue. The subject must not have a history of primary and secondary brain tumors. All subjects are evaluated for safety and all blood collections for pharmacokinetic analysis are collected as scheduled. All studies are performed with institutional ethics committee approval and patient consent.

**Phase I:** Patients receive the alkylating agent temozolomide orally on day 1 of the first cycle. On day 4 of the first cycle, patients receive oral treatment with the selective PARP inhibitor ABT-888, and i.v. treatment with the AP site binder methoxyamine. Beginning with the second cycle, which is four weeks long, the patients are dosed with ABT-888 and methoxyamine in cohorts of 3-6 patients in combination with a standard dose of oral temozolomide given on day 1-5 of the cycle at a dose of about 150 mg/kg. Patients receive methoxyamine and ABT-888 daily for a specific duration during the treatment cycle (e.g., oral treatment with the selective PARP inhibitor ABT-888 on days 1-7 at doses up to 40 mg bid, and i.v. treatment with the AP site binder methoxyamine on day 1 at doses up to 100 mg/kg). Doses of the selective PARP inhibitor ABT-888 and the AP site binder methoxyamine may be held or modified for toxicity. Treatment repeats every 28 days in the absence of unacceptable toxicity. Cohorts of patients receive fixed doses of the one BER pathway inhibitor (e.g., ABT-888) and escalating doses of the other BER pathway inhibitor (e.g., methoxyamine) until the maximum tolerated dose (MTD) for the combination is determined. The MTD is defined as the dose preceding that at which 2 of 3 or 2 of 6 patients experience dose-limiting toxicity. Dose limiting toxicities are determined in any suitable manner, e.g., according to the definitions and standards set by the National Cancer Institute (NCI) Common Terminology for Adverse Events (CTCAE) Version 3.0 (August 9, 2006).

**Phase II:** Patients receive treatment with the selective PARP inhibitor ABT-888, and the AP site binder methoxyamine as in phase I at the MTD determined in phase I. Treatment repeats every 4 weeks for 2-6 courses in the absence of disease progression or unacceptable toxicity. After completion of 2 courses of study therapy, the tumor is assessed by CT scan and patients who achieve a complete or partial response may receive additional courses. Patients who maintain stable disease for more than 2 months after completion of 6 courses of study therapy may receive an additional 6 courses at the time of disease progression, provided they meet original eligibility criteria.

Blood Sampling: Serial blood is drawn by direct vein puncture before and after administration of the methoxyamine and ABT-888. Venous blood samples (5 mL) for determination of serum concentrations are obtained at about 10 minutes prior to dosing and at specified times after dosing. Each serum sample is divided into two aliquots. All serum samples are stored at -20°C. Serum samples are shipped on dry ice.

Pharmacokinetics: Patients undergo plasma/serum sample collection for pharmacokinetic evaluation before beginning treatment and at specified days throughout the treatment cycle. Pharmacokinetic parameters are calculated by model independent methods on a Digital Equipment Corporation VAX 8600 computer system using the latest version of the BIOAVL software. The following pharmacokinetics parameters are determined: peak serum concentration (Cₘₐₓ); time to peak serum concentration (tₘₐₓ); area under the concentration-time curve (AUC) from time zero to the last blood sampling time (AUC₀₋₇₂) calculated with the use of the linear trapezoidal rule; and terminal elimination half-life (t_{1/2}), computed from the elimination rate constant. The elimination rate constant is estimated by linear regression of consecutive data points in the terminal linear region of the log-linear concentration-time plot. The mean, standard deviation (SD), and coefficient of variation (CV) of the pharmacokinetic parameters are calculated for each treatment. The ratio of the parameter means (preserved formulation/non-preserved formulation) is calculated.

### EXAMPLE 5: Method of Treatment - WBRT Combined with Methoxyamine and a PARP Inhibitor

Human Clinical Trial of the Safety and Pharmacokinetics of Whole Brain Radiation Therapy in Combination with Two BER Pathway Inhibitors in the Treatment of Cancer.

**Objective:** To determine the safety, tolerability, and pharmacokinetics of methoxyamine and the selective PARP inhibitor ABT-888 in combination with conventional whole brain radiation therapy (WBRT) in the treatment of subjects with solid tumors metastatic to the brain.

**Study Design:** This will be a Phase I, multi-center, open-label, non-randomized dose escalation study in cancer patients for the treatment of solid tumors metastatic to the brain. Patients present with histologically or cytologically confirmed non-CNS primary solid malignancy and pathologically or radiographically confirmed metastatic disease in the brain. WBRT should be clinically indicated with the exception of prophylactic treatment, and the patient has to have a Karnofsky Performance Status (KPS) greater than or equal to a score of 70. Patients should not have received prior treatment with WBRT or have had exposure to any study drug (e.g., PARP inhibitor and/or methoxyamine) prior to study entry. Patients must not have received treatment for their cancer within 14 days prior to the beginning of the trial. Treatments include the use of chemotherapy, immunotherapy, biologic therapy such as monoclonal antibodies, or investigational therapy. Patients must not display unresolved or unstable, serious toxicity from prior administration of another investigational drug and/or prior anticancer treatment. All subjects are evaluated for safety and all blood collections for pharmacokinetic analysis are collected as scheduled. All studies are performed with institutional ethics committee approval and patient consent.

**Phase I:** Patients receive Whole Brain Radiation Therapy (WBRT) as either 15 fractions of 2.5 Gy over three weeks to a total dose of 37.5 Gy or 10 fractions of 3 Gy over two weeks to a total dose of 30 Gy. Patients also receive oral treatment with the selective PARP inhibitor ABT-888 at doses up to 40 mg bid, and intravenous treatment with the AP site binder methoxyamine at doses up to 100 mg/kg daily during the WBRT treatment period. Doses of the selective PARP inhibitor ABT-888 and the AP site binder methoxyamine may be held or modified for toxicity. Treatment repeats every 28 days in the absence of unacceptable toxicity. Cohorts of patients receive fixed doses of the one BER pathway inhibitor (e.g., ABT-888) and escalating doses of the other BER pathway inhibitor (e.g., methoxyamine) until the maximum tolerated dose (MTD) for the combination is determined. The MTD is defined as the dose preceding that at which 2 of 3 or 2 of 6 patients experience dose-limiting toxicity. Dose limiting toxicities are determined in any suitable manner, e.g., according to the definitions and standards set by the National Cancer Institute (NCI) Common Terminology for Adverse Events (CTCAE) Version 3.0 (August 9, 2006).

Blood Sampling: Serial blood is drawn by direct vein puncture before and after administration of methoxyamine and ABT-888. Venous blood samples (5 mL) for determination of serum concentrations are obtained at about 10 minutes prior to dosing and at specified times after dosing. Each serum sample is divided into two aliquots. All serum samples are stored at -20°C. Serum samples are shipped on dry ice.

Pharmacokinetics: Patients undergo plasma/serum sample collection for pharmacokinetic evaluation before beginning treatment and at specified days throughout the treatment cycle. Pharmacokinetic parameters are calculated by model independent methods on a Digital Equipment Corporation VAX 8600 computer system using the latest version of the BIOAVL software. The following pharmacokinetics parameters are determined: peak serum concentration (Cₘₐₓ); time to peak serum concentration (tₘₐₓ); area under the concentration-time curve (AUC) from time zero to the last blood sampling time (AUC₀₋₇₂) calculated with the use of the linear trapezoidal rule; and terminal elimination half-life (t_{1/2}), computed from the elimination rate constant. The elimination rate constant is estimated by linear regression of consecutive data points in the terminal linear region of the log-linear concentration-time plot. The mean, standard deviation (SD), and coefficient of variation (CV) of the pharmacokinetic parameters are calculated for each treatment. The ratio of the parameter means (preserved formulation/non-preserved formulation) is calculated.

The examples and cases described herein are for illustrative purposes only and in some cases, various modifications or changes are to be included within the purview of disclosure and scoped of the appended claims.

## Claims

1. a) Methoxyamine;
b) veliparib; and
c) temozolomide;
for simultaneous, separate or sequential use in treating cancer, wherein the methoxyamine and veliparib potentiate the cytotoxic activity of the temozolomide.

2. The compounds according to claim 1 for use according to claim 1, wherein the cancer is lung cancer, non-small cell lung cancer, mesothelioma, brain cancer, glioblastoma multiforme, skin cancer, or melanoma.

3. The compounds according to claim 1 for use according to claims 1 or 2, wherein the cancer is melanoma.

4. The compounds according to claim 1 for use according to claim 3 wherein the melanoma is primary melanoma.

5. The compounds according to claim 1 for use according to claim 3 wherein the melanoma is metastatic melanoma.

6. The compounds according to claim 1 for use according to any of claims 1 to 3, wherein the methoxyamine, the veliparib, and the temozolomide are administered orally, intravenously, intraperitoneally, directly by injection to a tumor, topically, or a combination thereof.

7. The compounds according to claim 1 for use according to claim 6 wherein the methoxyamine is administered orally.

8. The compounds according to claim 1 for use according to claim 6 wherein the temozolomide is administered orally.

9. The compounds according to claim 1 for use according to any of claims 1 to 6, wherein the methoxyamine is administered at doses of 5 mg/m² per day to 100 mg/m² per day.

10. The compounds according to claim 1 for use according to any of claims 1 to 6, wherein the veliparib is administered at doses of 1 mg/kg per day to 50 mg/kg per day.

11. A pharmaceutical composition comprising:
a) methoxyamine;
b) veliparib; and
c) temozolomide;
wherein the methoxyamine and the veliparib potentiate the cytotoxic activity of the temozolomide.

12. The composition according to claim 11 for use in the treatment of lung cancer, non-small cell lung cancer, mesothelioma, brain cancer, glioblastoma multiforme, skin cancer, or melanoma.

13. The composition according to claim 11 for use in the treatment of melanoma.

## Patentansprüche

1. a) Methoxyamin;
b) Veliparib; und
c) Temozolomid;
zur gleichzeitigen, getrennten oder aufeinanderfolgenden Verwendung beim Behandeln von Krebs, wobei das Methoxyamin und das Veliparib die zytotoxische Aktivität des Temozolomids verstärken.

2. Verbindungen nach Anspruch 1 zur Verwendung nach Anspruch 1, wobei der Krebs Lungenkrebs, nicht-kleinzelliger Lungenkrebs, Mesotheliom, Gehirnkrebs, Glioblastoma multiforme, Hautkrebs oder Melanom ist.

3. Verbindungen nach Anspruch 1 zur Verwendung nach Anspruch 1 oder 2, wobei der Krebs Melanom ist.

4. Verbindungen nach Anspruch 1 zur Verwendung nach Anspruch 3, wobei das Melanom primäres Melanom ist.

5. Verbindungen nach Anspruch 1 zur Verwendung nach Anspruch 3, wobei das Melanom metastatisches Melanom ist.

6. Verbindungen nach Anspruch 1 zur Verwendung nach einem der Ansprüche 1 bis 3, wobei das Methoxyamin, das Veliparib und das Temozolomid oral, intravenös, intraperitoneal, direkt durch Injizieren in einen Tumor, topisch oder durch eine Kombination daraus verabreicht werden.

7. Verbindungen nach Anspruch 1 zur Verwendung nach Anspruch 6, wobei das Methoxyamin oral verabreicht wird.

8. Verbindungen nach Anspruch 1 zur Verwendung nach Anspruch 6, wobei das Temozolomid oral verabreicht wird.

9. Verbindungen nach Anspruch 1 zur Verwendung nach einem der Ansprüche 1 bis 6, wobei das Methoxyamin in Dosen von 5 mg/m² pro Tag bis 100 mg/m² pro Tag verabreicht wird.

10. Verbindungen nach Anspruch 1 zur Verwendung nach einem der Ansprüche 1 bis 6, wobei das Veliparib in Dosen von 1 mg/kg pro Tag bis 50 mg/kg pro Tag verabreicht wird.

11. Pharmazeutische Zusammensetzung, Folgendes umfassend:
a) Methoxyamin;
b) Veliparib; und
c) Temozolomid;
wobei das Methoxyamin und das Veliparib die zytotoxische Aktivität des Temozolomids verstärken.

12. Zusammensetzung nach Anspruch 11 zur Verwendung beim Behandeln von Lungenkrebs, nicht-kleinzelligem Lungenkrebs, Mesotheliom, Gehirnkrebs, Glioblastoma multiforme, Hautkrebs oder Melanom.

13. Zusammensetzung nach Anspruch 11 zur Verwendung beim Behandeln von Melanom.

## Revendications

1. a) Méthoxamine ;
b) véliparib ; et
c) témozolomide ;
pour une utilisation simultanée, séparée ou séquentielle dans le traitement du cancer, où la méthoxyamine et le véliparib potentialisent l'activité cytotoxique du témozolomide.

2. Composés selon la revendication 1 pour une utilisation selon la revendication 1, où le cancer est le cancer du poumon, le cancer du poumon non à petites cellules, le mésothéliome, le cancer du cerveau, le glioblastome multiforme, le cancer de la peau ou le mélanome.

3. Composés selon la revendication 1 pour une utilisation selon les revendications 1 ou 2, où le cancer est le mélanome.

4. Composés selon la revendication 1 pour une utilisation selon la revendication 3, où le mélanome est le mélanome primaire.

5. Composés selon la revendication 1 pour une utilisation selon la revendication 3, où le mélanome est le mélanome métastatique.

6. Composés selon la revendication 1 pour une utilisation selon l'une quelconque des revendications 1 à 3, où la méthoxyamine, le véliparib et le témozolomide sont administrés par voie orale, intraveineuse, intrapéritonéale, directement par injection dans une tumeur, topique, ou une combinaison de celles-ci.

7. Composés selon la revendication 1 pour une utilisation selon la revendication 6, où la méthoxyamine est administrée par voie orale.

8. Composés selon la revendication 1 pour une utilisation selon la revendication 6, où le témozolomide est administrée par voie orale.

9. Composés selon la revendication 1 pour une utilisation selon l'une quelconque des revendications 1 à 6, où la méthoxyamine est administrée à des doses de 5 mg/m² par jour à 100 mg/m² par jour.

10. Composés selon la revendication 1 pour une utilisation selon l'une quelconque des revendications 1 à 6, où le véliparib est administré à des doses de 1 mg/kg par jour à 50 mg/kg par jour.

11. Composition pharmaceutique comprenant :
a) de la méthoxyamine ;
b) du véliparib ; et
c) du témozolomide ;
où la méthoxyamine et le véliparib potentialisent l'activité cytotoxique du témozolomide.

12. Composition selon la revendication 11 pour une utilisation dans le traitement du cancer du poumon, du cancer du poumon non à petites cellules, du mésothéliome, du cancer du cerveau, du glioblastome multiforme, du cancer de la peau ou du mélanome.

13. Composition selon la revendication 11 pour une utilisation dans le traitement du mélanome.
